# EUROPÄISCHE PATENTSCHRIFT

(11) **EP 2 714 640 B1**
(45) Veröffentlichungstag und Bekanntmachung des Hinweises auf die Patenterteilung: **29.08.2018**
(21) Anmeldenummer: 12716417.6
(22) Anmeldetag: 24.04.2012
(51) Int. Cl.: C07C 51/09, C07C 57/04, C07C 67/20, C07C 231/06

(54) **VERFAHREN ZUR HERSTELLUNG VON METHACRYLSÄURE**
PROCESS FOR PREPARING METHACRYLIC ACID
PROCÉDÉ DE PRÉPARATION D'ACIDE MÉTHACRYLIQUE

(30) Priorität: 27.05.2011 DE 102011076642
(43) Veröffentlichungstag der Anmeldung: 09.04.2014
(73) Patentinhaber: Evonik Röhm GmbH, 64293 Darmstadt (DE)
(72) Erfinder: BRÖLL, Dirk, 63225 Langen (DE); SIEGERT, Hermann, 64342 Seeheim-Jugenheim (DE); HILTNER, Horst, 48159 Münster (DE); KRAUSS, Thomas, 64404 Bickenbach (DE)
(86) Internationale Anmeldenummer: PCT/EP2012/057421
(87) Internationale Veröffentlichungsnummer: WO 2012/163600

(56) Entgegenhaltungen:
- EP-A1- 1 813 586
- US-B1- 7 253 307

## Beschreibung

Die vorliegende Erfindung betrifft ein Verfahren zur Herstellung von Methacrylsäure auf Basis der Hydrolyse von Methacrylsäureestern.

Aus dem Stand der Technik ist eine Vielzahl von Verfahren zur Herstellung von Methacrylsäure bekannt.

Eine übliche Verfahrensweise besteht in der kontrollierten Oxidation von Kohlenwasserstoffgasen, beispielsweise Propylen oder Butylen. Nachteilig an diesen Verfahren sind die insgesamt gesehen eher geringen Ausbeuten, die hierdurch erhalten werden.

Darüber hinaus kann Methacrylsäure durch die Umsetzung von Methacrylamid mit Wasser erhalten werden. Dieses Verfahren wird insbesondere in US 7,253,307 beschrieben. Gemäß dieser Druckschrift kann die Umsetzung des Methacrylamids mit Wasser in einem Rührkesselreaktor oder einem Röhrenreaktor erfolgen. Vorzugsweise wird die Reaktion bei einem Druck im Bereich von 3,65 bis 7,70 bar und einer Temperatur im Bereich von 50 bis 210 °C durchgeführt.

Die in US 7,253,307 beschriebenen Verfahren zur Herstellung von Methacrylsäure führen bereits zu guten Ausbeuten bei einer hohen Reinheit. Allerdings stellt Methacrylsäure ein wichtiges Erzeugnis der chemischen Industrie dar, welches als Ausgangsstoff für viele wichtige Produkte dient. Daher ist eine maximale Ausbeute und eine besonders hohe Reinheit bei geringen Herstellungskosten wesentlich für den wirtschaftlichen Erfolg eines solchen Herstellungsprozesses. Schon relativ kleine Verbesserungen hinsichtlich der Ausbeuten, der Standzeiten der Anlagen oder ähnlicher Verfahrensmerkmale führen zu einem bedeutenden Fortschritt hinsichtlich der Abfallmengen und der Herstellkosten.

Ebenso kann α-Hydroxyisobuttersäure als Ausgangsstoff zur Herstellung von Methacrylsäure dienen. Ein derartiges Verfahren wird zum Beispiel in US 3,487,101 beschrieben, wo die Herstellung diverser Methacrylsäurederivate, insbesondere Methacrylsäure und Methacrylsäureester, ausgehend von 2-Hydroxyisobuttersäure (HIBS) in der Flüssigphase, dadurch gekennzeichnet ist, dass die Umsetzung von HIBS zu Methacrylsäure in Gegenwart eines gelösten basischen Katalysators bei hohen Temperaturen zwischen 180- 320 °C in Gegenwart hochsiedender Ester (z.b. Phthalsäuredimethylester) und inneren Anhydriden (z.B. Phthalsäureanhydrid) durchgeführt wird. Laut Patent werden bei HIBS Umsätzen von > 90 % MAS-Selektivitäten um 98 % erreicht. Über die Langzeitstabilität der flüssigen Katalysatorlösung insbesondere der Erschöpfung des eingesetzten Anhydrids werden keine Angaben gemacht.

DE-OS 1 191 367 betrifft die Herstellung von Methacrylsäure (MAS) ausgehend von HIBS in der Flüssigphase, dadurch gekennzeichnet, dass die Umsetzung von HIBS zu Methacrylsäure in Gegenwart von Polymerisationsinhibitoren (wie z.B. Kupferpulver) und in Gegenwart eines Katalysatorgemischs bestehend aus Metallhalogeniden und Alkalihalogeniden bei hohen Temperaturen zwischen 180 - 220 °C durchgeführt wird. Laut Patent werden bei HIBS Umsätze > 90 % und MAS-Selektivitäten von > 99 % erreicht. Die besten Ergebnisse werden mit Katalysatormischungen aus Zinkbromid und Lithiumbromid erreicht. Es ist allgemein bekannt, dass die Verwendung halogenid-haltiger Katalysatoren bei hohen Temperaturen drastische Forderungen an die zu verwendenden Werkstoffe stellt und diese Probleme bezüglich der im Destillat befindlichen, verschleppten halogenierten Nebenprodukte auch in nachfolgenden Anlagenteilen auftreten.

EP 0 487 853 beschreibt die Herstellung von Methacrylsäure ausgehend von Acetoncyanhydrin (ACH), dadurch gekennzeichnet, dass man im ersten Schritt ACH mit Wasser bei moderaten Temperaturen in Gegenwart eines heterogenen Hydrolysekatalysators umsetzt und man im zweiten Schritt α-Hydroxyisobuttersäureamid mit Methylformiat oder Methanol/Kohlenmonoxid unter Entstehung von Formamid und Hydroxyisobuttersäuremethylester (HIBSM) umsetzt, und man im dritten Schritt HIBSM in Gegenwart eines heterogenen Ionenaustauschers mit Wasser zu HIBS verseift, und man im vierten Schritt HIBS dehydratisiert, indem man in flüssiger Phase bei hohen Temperaturen in Gegenwart eines löslichen Alkalisalzes reagieren lässt. Die Methacrylsäure-Herstellung ex HIBS wird bei hohen Umsätzen um 99 % mit mehr oder weniger quantitativen Selektivitäten beschrieben. Die Vielzahl der notwendigen Reaktionsschritte und die Notwendigkeit der Zwischenisolierung einzelner Intermediate, insbesondere auch die Durchführung einzelner Prozessschritte bei erhöhtem Druck, machen das Verfahren kompliziert und damit letztlich unwirtschaftlich. Des Weiteren fällt zwingend Formamid an, wobei diese Verbindung vielfach als unerwünschtes Nebenprodukt angesehen wird, welches teuer entsorgt werden muss.

DE-OS 1 768 253 beschreibt ein Verfahren zur Herstellung von Methacrylsäure durch Dehydratisierung von HIBS, dadurch gekennzeichnet, dass man HIBS in flüssiger Phase bei einer Temperatur von wenigstens 160 °C in Gegenwart eines Dehydratisierungskatalysators umsetzt, der aus einem Metallsalz von HIBS besteht. Besonders geeignet sind in diesem Fall die Alkali- und Erdalkalisalze von HIBS, die in einer HIBS-Schmelze durch Umsetzung geeigneter Metallsalze in situ hergestellt werden. Laut Patent werden MAS Ausbeuten bis 95 % ex HIBS beschrieben, wobei der Feed der kontinuierlichen Verfahrensweise ex HIBS und ca. 1,5 Gew.-% HIBS-Alkalisalz besteht.

RU 89631 betrifft ein Verfahren zur Herstellung von Methacrylsäure ausgehend von HIBS durch Wasserabspaltung in flüssiger Phase, dadurch gekennzeichnet, dass die Reaktion in Abwesenheit eines Katalysators mit einer wässrigen Lösung von HIBS (bis 62 Gew.-% HIBS in Wasser) unter Druck bei hohen Temperaturen 200°C - 240°C durchgeführt wird.

Intensiv untersucht wurde darüber hinaus die Verwendung von Propen als Basisrohstoff, wobei man über die Stufen Hydrocarbonylierung zur Isobuttersäure und dehydrierender Oxidation in moderaten Ausbeuten zur Methacrylsäure gelangt.

Es ist bekannt, Propanal oder Propionsäure, die in technischen Prozessen ausgehend von Ethylen und C-1 Bausteinen wie Kohlenmonoxid zugänglich sind, als Basisrohstoff einzusetzen. In diesen Prozessen wird in einer aldolisierenden Reaktion mit Formaldehyd unter Dehydratisierung der in situ entstehenden β-Hydroxycarbonylverbindung zur entsprechenden a, β-ungesättigten Verbindung umgesetzt. Eine Übersicht über die gängigen Verfahren zur Herstellung der Methacrylsäure und deren Ester findet sich in der Literatur wie z.B. Weissermel, Arpe "Industrielle organische Chemie", VCH, Weinheim 1994, 4. Auflage, S.305 ff oder Kirk Othmer "Encyclopedia of Chemical Technology", 3. Ausgabe, Vol. 15, Seite 357.

Aufgabe der Erfindung ist es daher ein neues Verfahren zur Herstellung von Methacrylsäure zur Verfügung zu stellen, dass die genannten Nachteile nicht aufweist, insbesondere geringere Mengen Abfallsäure erzeugt, mit einem geringeren Energieverbrauch auskommt, sowie höhere Ausbeuten und einen geringeren Wassergehalt im Endprodukt ermöglicht.

Gelöst wird die Aufgabe durch ein Verfahren zur Herstellung von Methacrylsäure aufweisend folgende Schritte:
a) Bereitstellung von Acetoncyanhydrin
b) Umsetzung von Acetoncyanhydrin zu Methacrylamid
c) Veresterung von Methacrylamid in Gegenwart von Alkanolen zum entsprechenden Methacrylsäureester
d) Hydrolyse des Methacrylsäureesters zu Methacrylsäure mittels heterogener Katalysatoren und einem Methacrylsäureester/H₂O Verhältnis des Eduktstromes zwischen 0,5 und 5.
Überraschend wurde gefunden, dass mit dem erfindungsgemäßen Verfahren eine großtechnisch einfach realisierbare Methacrylsäuresynthese zur Verfügung gestellt werden kann. Das Verfahren zeichnet sich durch die oben genannten Vorteile sowie durch ein geringes Nebenproduktspektrum aus. Die erhaltene Methacrylsäure weist Reinheiten von ≥ 99,5 % auf.

Es wurde gefunden, dass mit dem Einsatz heterogener Katalysatoren im Hydrolyse-Schritt d) eine Trennstufe zur Abtrennung des Katalysators entfällt, sowie auf den Einsatz von Schwefelsäure verzichtet werden kann, was die damit verbundenen Korrosionsprobleme minimiert und das Anfallen von Abfallsäure verhindert.

Außerdem wurde gefunden, dass bezogen auf den ACH-Einsatz eine höhere Ausbeute an Methacrylsäure erzielt wird.

Die Darstellung von Acetoncyanhydrin aus Cyanwasserstoff und Aceton ist bekannt und beispielsweise in EP 1 171 420 und DE 102006058250 beschrieben. Die anschließende Umsetzung von Acetoncyanhydrin zu Methacrylamiden ist beispielsweise in WO 2008/068064 beschrieben.

Die Veresterung von Methacrylamid in Gegenwart von Methanol zu Methylmethacrylat ist ebenfalls Stand der Technik und beispielsweise in WO 2008/068063 beschrieben.

### Schritt a) Darstellung von Acetoncyanhydrin

Acetoncyanhydrin, α-Hydroxy-Isobutyronitril, ist das wichtigste Ausgangsprodukt für alle Derivate der Methacrylsäure und vor allem deren Ester. Technisch wird Acetoncyanhydrin durch basisch katalysierte Addition von Cyanwasserstoff (Blausäure) an Aceton hergestellt. In neutralem und besonders alkalischem Bereich steht Acetoncyanhydrin im Gleichgewicht mit seinen Ausgangskomponenten. Die kommerziellen Verfahren benutzen überwiegend Flüssigphasenprozesse, die sowohl diskontinuierlich als auch kontinuierlich in Gegenwart von Katalysatoren, wie Natronlauge, Kalilauge, Kaliumcarbonat, Natriumacetat/Essigsäure, Pyridin/Essigsäure sowie Anionenaustauscherharzen bei Temperaturen unterhalb 40 °C durchgeführt werden. Beispielhaft wird auf das Verfahren der Rohm und Haas hingewiesen (siehe Ullmann's Encyclopedia of Industrial Chemistry 5th ed. (1985), Seiten 91-92). In diesem Verfahren werden flüssige Blausäure, Aceton und ein basischer Katalysator kontinuierlich in einen Reaktor eingeführt und anschließend das Reaktionsgemisch nach Stabilisierung mit Schwefelsäure und Filtration des Katalysators durch eine zweistufige Destillation zunächst von nicht umgesetzter Blausäure und Aceton und dann von Wasser befreit. Die Abgase aus der ersten Destillationsstufe werden in den Reaktor rezykliert, reines stabilisiertes Acetoncyanhydrin am Sumpf der zweiten Destillationsstufe entnommen. Nachteilig an diesem Verfahren ist, dass aus einem Cyanwasserstoff enthaltenden Gas, beispielsweise einem Rohgas aus dem BMA- oder Andrussow-Prozess zur Herstellung von Cyanwasserstoff, Cyanwasserstoff verflüssigt werden muss.

Es ist auch bekannt, anstelle von verflüssigter Blausäure ein Cyanwasserstoff und inerte Gase enthaltendes Gasgemisch, beispielsweise Kokereigase, in einem Verfahren zur Herstellung von Acetoncyanhydrin einzusetzen. Im Verfahren der Reinpreussen AG (siehe Ullmann's Encyklopedie der Technischen Chemie, 4. Auflage, Band 7, Seiten 34-35) werden Cyanwaserstoff enthaltende Kokereigase nach einer Pottaschewäsche kontinuierlich im Gegenstrom mit Aceton, das 10 % Wasser enthält, gewaschen und die Reaktion zu Acetoncyanhydrin in Gegenwart eines basischen Katalysators in zwei hintereinander geschalteten Gaswaschkolonnen durchgeführt; die Aufarbeitung des Acetoncyanhydrin enthaltenden Reaktionsgemischs umfasst zwei Aceton-Kolonnen und zwei Kolonnen zur Reinigung des Acetoncyanhydrins.

Aus EP 1 171 420 ist ein Verfahren zur kontinuierlichen Herstellung von Acetoncyanhydrin bekannt, umfassend Umsetzung von Cyanwasserstoff mit Aceton in Gegenwart eines basischen Katalysators und Acetoncyanhydrin in einem Gas-Flüssig-Reaktor, welchem ein Cyanwasserstoff und inerte Gase enthaltendes Gasgemisch und Aceton kontinuierlich zugeführt und aus welchem eine Acetoncyanhydrin enthaltende flüssige Phase und eine die inerten Gase enthaltende Gasphase abgeführt werden, und destillative Abtrennung flüchtiger Bestandteile aus der flüssigen Phase, das dadurch gekennzeichnet ist, dass man die aus dem Reaktor abgeführte Gasphase in einem mit einem bei 100 bis 200 °C (Normaldruck) siedenden Lösungsmittel oder mit stabilisiertem reinen Acetoncyanhydrin beaufschlagten Gaswäscher von nicht umgesetztem Cyanwasserstoff und Aceton befreit, und die erhaltene Waschphase dem Gas-Flüssig-Reaktor zuführt. In diesem Verfahren wird vorzugsweise ein BMA-Rohgas oder ein Andrussow-Rohgas eingesetzt. Das aus den genannten üblichen Verfahren zur Herstellung von Cyanwasserstoff resultierende Gasgemisch kann als solches oder nach einer Säurewäsche verwendet werden. Das Rohgas aus dem BMA-Prozess, bei welchem aus Methan und Ammoniak im wesentlichen Blausäure und Wasserstoff gebildet werden, enthält gemäß Ullmanns's Encyclopedia of Technical Chemistry, 5th ed. (1987), Vol. A8, S. 161-163, typischerweise 22,9 Vol.-% HCN, 71,8 Vol.-% H₂, 2,5 Vol.-% NH₃, 1,1 Vol.-% N₂ und 1,7 Vol.-% CH₄.

Im bekannten Andrussow-Prozess werden aus Methan, Ammoniak und Luftsauerstoff Blausäure und Wasser gebildet. Das Rohgas des Andrussow-Prozesses enthält bei Einsatz von Luftsauerstoff als Sauerstoffquelle gemäß dem zuvor zitierten Dokument typischerweise 8 Vol.-% HCN, 22 Vol.-% H₂, 46,5 Vol.-% N₂, 15 Vol.-% H₂O, 5 Vol.-% CO, 2,5 Vol.-% NH₃ und je 0,5 Vol.-% CH₄ und CO₂.

Die Aufarbeitung des aus dem Reaktor abgezogenen Roh-Acetoncyanhydrins (Roh-ACH) erfolgt in dem Fachmann bekannter Weise. Zunächst wird das Roh-ACH durch Zugabe einer Säure stabilisiert, sodann werden die Leichtsieder HCN, Aceton und Wasser ein- oder mehrstufig abdestilliert. Eine zweckmäßige Ausgestaltung zur Aufarbeitung des Roh-ACH folgt aus EP 0 421 237.

Im Rahmen eines weiteren Verfahrenselementes, das im Zusammenhang mit der vorliegenden Erfindung eingesetzt werden kann, kann Acetoncyanhydrin, das in einer vorgelagerten Stufe beispielsweise aus der Umsetzung von Aceton mit Blausäure erhalten wurde, einer destillativen Aufarbeitung unterzogen werden.

### Schritt b) Umsetzung von Acetoncynhydrin zu Methacrylamid

Im Rahmen eines weiteren Verfahrensschritts wird das im ersten Schritt hergestellte Acetoncyanhydrin einer Hydrolyse unterzogen. Dabei bildet sich bei verschiedenen Temperaturstufen nach einer Reihe von Reaktionen als Produkt Methacrylamid.

Die Umsetzung wird in einer dem Fachmann bekannten Weise durch eine Reaktion zwischen konzentrierter Schwefelsäure und Acetoncyanhydrin bewirkt. Die Umsetzung ist exotherm, so dass beispielsweise zur Reaktionskontrolle Reaktionswärme aus dem System abgeführt werden kann.

### Schritt c) Veresterung von Methacrylamid in Gegenwart von Methanol zu Methylmethacrylat

Ein weiterer erfindungsgemäßer Schritt ist die Alkoholyse von Methacrylamid zu den entsprechenden Methacrylsäureestern. Grundsätzlich eignen sich hierzu beliebige Alkanole mit 1 bis 4 Kohlenstoffatomen, die linear oder verzweigt, gesättigt oder ungesättigt sein können, wobei Methanol besonders bevorzugt ist. Ebenso können diese Alkanole zusammen mit Methacrylsäureestern eingesetzt werden, was insbesondere bei Umesterungen der Fall ist. Die Menge an Amidlösung und an Alkanol wird derart geregelt, dass ein Gesamtmolverhältnis von Amid zu Alkanol von 1:1,4 bis 1:1,6 herrscht. Das Alkanol kann auf die Kesselkaskade derart verteilt werden, dass im ersten Reaktor das Molverhältnis 1:1,1 bis 1:1,4 beträgt und in den folgenden Reaktionsstufen bezogen auf den Gesamtamidstrom Molverhältnisse von 1:0,05 bis 1:0,3 eingestellt werden. Das in die Veresterung zugeführte Alkanol kann sich aus "Frischalkanol" sowie Alkanol aus Recyclingströmen der Aufarbeitungsstufen und bei Bedarf auch aus Recyclingströmen der Downstreamprozesse des Produktionsverbundes zusammensetzen.

Da Methacrylsäureester eine starke Neigung zur Polymerisation aufweisen, ist es vorteilhaft, Sorge zu tragen, dass eine derartige Polymerisation verhindert wird.

Um die Polymerisation zu verhindern, kann es vorteilhaft sein, eine Optimierung des Stoffflusses durchzuführen. Einerseits kann die Strömungsgeschwindigkeit des Methacrylsäureesters optimiert werden. Darüber hinaus kann es vorteilhaft sein, den Strom an Methacrylsäureester derart mit geeigneten Stabilisatoren zu versetzen, dass eine Polymerisation weitgehend unterdrückt wird.

Das im Rahmen der Veresterung und der anschließenden Vorreinigung erhaltene MMA bzw. der erhaltene Methacrylsäureester werden anschließend einer weiteren Behandlung zugeführt. Aus der Veresterung resultiert als verbleibender Reststoff verdünnte Schwefelsäure, die ebenfalls einer weiteren Verwertung zugeführt werden kann.

### Aufreinigung des Methacrylsäureesters

Grundsätzlich kann rohe Methacrylsäure oder ein roher Methacrylsäureester einer weiteren Reinigung unterzogen werden, um zu einem möglichst reinen Produkt zu gelangen. Dieser alternative Verfahrensschritt zur Reinigung kann beispielsweise einstufig sein. Es hat sich jedoch in vielen Fällen als vorteilhaft herausgestellt, wenn eine solche Reinigung mindestens zwei Stufen umfasst. Nach einer Vorreinigung durch Abtrennung der niedrigsiedenden Bestandteile empfiehlt sich eine anschließende Hauptreinigung mittels Destillation zur Abtrennung der hochsiedenden Bestandteile.

### Schritt d) Hydrolyse des Methacrylsäureester zu Methacrylsäure

Ein weiterer erfindungsgemäßer Verfahrensschritt ist die Hydrolyse des zuvor dargestellten Methacrylsäureester zu Methacrylsäure. Die Edukte dieses Verfahrensschrittes (Methacrylsäureester und Wasser) werden zusammengeführt und die Reaktionsmischung auf die geeignete Temperatur gebracht. Die Reaktionsbedingungen hinsichtlich Druck und Temperatur sind in dem Fachmann bekannter Weise an die Alkoholkomponente des eingesetzten Methacrylsäureesters anzupassen. Besonders bevorzugt wird im erfindungsgemäßen Verfahren Methylmethacrylat eingesetzt. Die Hydrolyse erfolgt in Anwesenheit von heterogenen Katalysatoren. Geeignete Katalysatoren werden ausgewählt aus der Gruppe der Zeolithe, lonenaustauscherharze und amorphen Säurekatalysatoren. Eine Vielzahl geeigneter Katalysatoren findet der Fachmann in EP 1 352 891. Besonders bevorzugt sind kationische lonenaustauscherharze. Geeignete Katalysatoren sind Ionenaustauscher wie Lewatit K1221, der Fa. Lanxess AG, Lewatit K2629, der Fa. Lanxess AG, Dowex CM-4, der Fa. Dow Chemical, Dowex M-31, der Fa. Dow Chemical, Dowex M-3 MS, der Fa. Dow Chemical, Amberlyst 39 Wet, der Fa. Rohm & Haas, Amberlyst CSP2, der Fa. Rohm & Haas, Amberlyst CSP3, der Fa. Rohm & Haas, DIAION PK208, der
Fa. Mitsubishi Chemicals, DIAION PK216, der Fa. Mitsubishi Chemicals, DIAION PK228, der Fa. Mitsubishi Chemicals. Ein ganz besonders bevorzugter Katalysator ist Lewatit K2431, der Fa. Lanxess AG.

Es wurde gefunden, dass in Abhängigkeit von der Polymerisationsneigung des eingesetzten Monomergemisches, der Art des Katalysators und/oder der Größe des Katalysatorbettes, die Durchströmung des Katalysatorbettes von oben oder unten vorteilhaft ist. Bevorzugt wird von unten angeströmt.

Die Hydrolyse der Methacrylsäureester wird bei Temperaturen zwischen 50 und 200°C, bevorzugt zwischen 70 und 150°C, besonders bevorzugt bei 90-120°C und ganz besonders bevorzugt bei 100 bis 110°C durchgeführt.

Vorzugsweise wird gleichzeitig bei Überdruck, bevorzugt unter einem Druck von 0,1-9 bar Überdruck, besonders bevorzugt bei 2-4 bar Überdruck gearbeitet. Der Druck wird im Reaktor so eingestellt, dass dieser Druck am Reaktorausgang gemessen wird.

Der Eduktstrom Methacrylsäureester und Wasser hat eine Zusammensetzung, bei der das Verhältnis Methacrylsäureester zu Wasser zwischen 0,5 und 5 liegt, bevorzugt zwischen 1 und 4, besonders bevorzugt zwischen 1,5 und 3.

Die Verweilzeit (auf Basis des Katalysator-Leerraumvolumens berechnet) beträgt 10-120 min, bevorzugt 30-90 min, besonders bevorzugt 45-75 min.

### Verfahrensbeschreibung

### Schritt a) Bereitstellung von Acetoncyanhydrin

Die Bereitstellung von Acetoncyanhydrin wird nach allgemein bekannten Verfahren (siehe beispielsweise Ullmanns Enzyklopädie der technischen Chemie, 4. Auflage, Band 7) durchgeführt. Es werden dabei als Reaktionspartner Aceton und Blausäure eingesetzt. Bei der Umsetzung handelt es sich um eine exotherme Reaktion. Um einer Zersetzung des im Rahmen dieser Reaktion gebildeten Acetoncyanhydrins entgegenzuwirken, wird üblicherweise die Reaktionswärme durch eine geeignete Vorrichtung abgeführt. Die Umsetzung kann dabei grundsätzlich als Batchprozess oder als kontinuierliches Verfahren geführt werden, sofern eine kontinuierliche Fahrweise bevorzugt ist, wird die Umsetzung häufig in einem Schlaufenreaktor, der entsprechend eingerichtet ist, durchgeführt.

Ein Hauptmerkmal einer in hohen Ausbeuten zum gewünschten Produkt führenden Fahrweise liegt darin, dass bei ausreichender Reaktionszeit das Reaktionsprodukt gekühlt wird und das Reaktionsgleichgewicht in Richtung des Reaktionsproduktes verschoben wird. Darüber hinaus wird das Reaktionsprodukt zum Vorteil der Gesamtausbeute häufig mit einem entsprechenden Stabilisator versetzt, um bei der späteren Aufarbeitung einer Zersetzung in die Ausgangsstoffe vorzubeugen.

Die Vermischung der Reaktionspartner Aceton und Blausäure kann grundsätzlich auf beliebige Weise erfolgen. Die Art der Vermischung hängt insbesondere davon ab, ob eine diskrete Fahrweise, beispielsweise im Batchreaktor, oder eine kontinuierliche Fahrweise, beispielsweise im Schlaufenreaktor, gewählt wird.

Grundsätzlich kann es vorteilhaft sein, wenn das Aceton in die Reaktion über einen Vorlagebehälter eingespeist wird, der über einen Waschturm verfügt. Entlüftungsleitungen, die Aceton und Blausäure enthaltende Abluft führen, können so beispielsweise durch diesen Vorlagebehälter geführt werden. Im Waschturm, der an den Vorlagebehälter angeschlossen ist, kann die aus dem Vorlagebehälter entweichende Abluft mit Aceton gewaschen werden, wodurch Blausäure aus der Abluft entfernt und in den Prozess rückgeführt wird. Hierzu wird beispielsweise ein Teil der vom Vorlagebehälter in die Reaktion eingeführten Acetonmenge im Teilstrom über einen Kühler, vorzugsweise über einen Sole-Kühler, in den Kopf des Waschturms geführt und so das gewünschte Ergebnis erzielt.

Je nach Umfang der zu produzierenden Menge an Endprodukten kann es vorteilhaft sein, dass Aceton aus mehr als nur einem Vorlagebehälter der Reaktion zuzuführen. Dabei kann jeder der zwei oder mehr Vorlagebehälter einen entsprechenden Waschturm tragen. Es ist jedoch in vielen Fällen ausreichen, wenn nur einer der Vorlagebehälter mit einem entsprechenden Waschturm ausgerüstet ist. In diesem Fall ist es jedoch oft sinnvoll, wenn entsprechende Abluft führende Leitungen, die Aceton und Blausäure transportieren können, über diesen Behälter bzw. über diesen Waschturm geführt werden.

Die Temperatur des Acetons im Vorlagebehälter kann grundsätzlich innerhalb eines im Wesentlichen beliebigen Bereiches liegen, insofern das Aceton sich bei der entsprechenden Temperatur im flüssigen Zustand befindet. Vorteilhafterweise beträgt die Temperatur im Vorlagebehälter jedoch 0 bis 20 °C.

Im Waschturm wird das zum Waschen eingesetzte Aceton über einem entsprechenden Kühler, beispielsweise über einen Plattenkühler mit Sole auf eine Temperatur von 0 bis 10 °C gekühlt. Die Temperatur des Acetons beim Eintritt in den Waschturm beträgt daher beispielsweise 2 bis 6 °C.

Die im Rahmen der Reaktion benötigte Blausäure kann entweder in flüssiger oder in gasförmiger Form in den Reaktor eingeführt werden. Es kann sich dabei beispielsweise um Rohgas aus dem BMA- oder aus dem Andrussow-Prozess handeln.

Der Cyanwasserstoff kann beispielsweise verflüssigt werden, beispielsweise durch den Einsatz einer entsprechenden Kühlsole. Anstelle von verflüssigter Blausäure kann Kokereigas eingesetzt werden. So werden beispielsweise Cyanwasserstoff enthaltende Kokereigase nach einer Wäsche mit Pottasche kontinuierlich im Gegenstrom mit Aceton, das 10 % Wasser enthält, gewaschen und die Reaktion zu Acetoncyanhydrin kann in Gegenwart eines basischen Katalysators in zwei hintereinander geschalteten Gaswaschkolonnen durchgeführt werden.

Im Rahmen einer weiteren Ausführungsform kann ein Cyanwasserstoff und inerte Gase enthaltendes Gasgemisch, insbesondere ein Rohgas aus dem BMA- oder aus dem Andrussow-Prozess mit Aceton in Gegenwart eines basischen Katalysators und Acetoncyanhydrin in einem Gas-Flüssig-Reaktor umgesetzt werden.

Im Rahmen des hier beschriebenen Verfahrens wird vorzugsweise ein BMA-Rohgas oder ein Andrussow-Rohgas eingesetzt. Dass aus den oben genannten üblichen Verfahren zur Herstellung von Cyanwasserstoff resultierende Gasgemisch kann als solches oder nach einer Säurewäsche verwendet werden. Das Rohgas aus dem BMA-Prozess, bei welchem aus Methan und Ammoniak im Wesentlichen Blausäure und Wasserstoff gebildet werden, enthält typischerweise 22,9 Vol.-% HCN, 71,8 Vol.-% H₂, 2,5 Vol.-% NH₃, 1,1 Vol.-% N₂, 1,7 Vol.-% CH₄. Im bekannten Andrussow-Prozess werden aus Methan und Ammoniak und Luftsauerstoff Blausäure und Wasser gebildet. Das Rohgas des Andrussow-Prozesses enthält beim Einsatz von Sauerstoff als Sauerstoffquelle typischerweise 8 Vol.-% HCN, 22 Vol.-% H₂, 46,5 Vol.-% N₂, 15 Vol.-% H₂O, 5 Vol.-% CO, 2,5 Vol.-% NH₃ und je 0,5 Vol.-% CH₄ und CO₂.

Beim Einsatz eines nicht säuregewaschenen Rohgases aus dem BMA- oder Andrussow-Prozess wirkt der im Rohgas enthaltene Ammoniak häufig als Katalysator für die Reaktion. Da der im Rohgas enthaltene Ammoniak häufig die als Katalysator erforderliche Menge übersteigt und deshalb zu hohen Verlusten an zur Stabilisierung eingesetzter Schwefelsäure führen kann, wird ein derartiges Rohgas oft einer Säurewäsche unterzogen, um Ammoniak daraus zu eliminieren. Beim Einsatz eines solchen, mit Säure gewaschenen Rohgases muss dann allerdings ein geeigneter basischer Katalysator dem Reaktor in katalytischer Menge zugesetzt werden. Im Prinzip können dabei bekannte anorganische oder organische basische Verbindungen als Katalysator fungieren.

Cyanwasserstoff in gasförmiger oder in flüssiger Form bzw. ein Cyanwasserstoff enthaltendes Gasgemisch und Aceton werden im Rahmen der kontinuierlichen Fahrweise fortlaufend einem Schlaufenreaktor zugeführt. Der Schlaufenreaktor umfasst dabei mindestens eine Möglichkeit zum Zuführen von Aceton oder zwei oder mehr solcher Möglichkeiten, mindestens eine Möglichkeit zur Zuführung von flüssiger oder gasförmiger Blausäure, oder zwei oder mehr solcher Möglichkeiten sowie mindestens eine Möglichkeit zum Zuführen eines Katalysators.

Als Katalysator eignen sich grundsätzlich beliebige alkalische Verbindungen wie Ammoniak, Natronlauge oder Kalilauge, die die Umsetzung von Aceton und Blausäure zu Acetoncyanhydrin katalysieren können. Es hat sich jedoch als vorteilhaft herausgestellt, wenn als Katalysator ein organischer Katalysator, insbesondere ein Amin eingesetzt wird. Geeignet sind beispielsweise sekundäre oder tertiäre Amine, wie Diethylamin, Dipropylamin, Triethylamin, Tri-n-propylamin und dergleichen.

Ein im Rahmen des beschriebenen Verfahrenselementes einsetzbarer Schlaufenreaktor weist darüber hinaus noch mindestens eine Pumpe, oder zwei oder mehr Pumpen, und mindestens eine Mischvorrichtung, oder zwei oder mehr solcher Mischvorrichtungen auf.

Als Pumpe eignen sich grundsätzlich alle Pumpen, die geeignet sind, den Umlauf des Reaktionsgemisches im Schlaufenreaktor zu gewährleisten.

Als Mischvorrichtungen sind sowohl Mischvorrichtungen mit beweglichen Elementen als auch so genannte Statikmischer geeignet, bei denen unbewegliche Strömungswiderstände vorgesehen sind. Entsprechende Mischer können aus Kunststoff oder Metall bestehen. Als Kunststoff eignen sich beispielsweise PVC, PP, HDPE, PVDF, PFA oder PTFE. Metallmischer können beispielsweise aus Nickellegierungen, Zirkonium, Titan und dergleichen bestehen. Ebenfalls geeignet sind beispielsweise Rechteckmischer.

Die Zugabe des Katalysators erfolgt vorzugsweise im Schlaufenreaktor nach der Pumpe und vor einem im Schlaufenreaktor vorhandenen Mischelement. Katalysatoren werden im Rahmen der beschriebenen Reaktion beispielsweise in einer solchen Menge eingesetzt, dass die Gesamtreaktion bei einem pH-Wert von maximal 8, insbesondere maximal 7,5 oder 7 gefahren wird. Es kann bevorzugt sein, wenn der pH-Wert bei der Reaktion sich innerhalb eines Rahmens von 6,5 bis 7,5, beispielsweise 6,8 bis 7,2 bewegt.

Es ist im Rahmen des beschriebenen Verfahrens alternativ zur Zugabe des Katalysators in den Schlaufenreaktor nach der Pumpe und vor einer Mischvorrichtung auch möglich, den Katalysator zusammen mit dem Aceton in den Schlaufenreaktor einzuspeisen. In einem solchen Fall kann es vorteilhaft sein, wenn für eine entsprechende Vermischung von Aceton und Katalysator vor Einspeisung in den Schlaufenreaktor gesorgt wird. Eine entsprechende Vermischung kann beispielsweise durch den Einsatz eines Mischers mit beweglichen Teilen oder durch Einsatz eines Statikmischers erfolgen.

Wenn im Rahmen des beschriebenen Verfahrens eine kontinuierliche Fahrweise in einem Schlaufenreaktor als Betriebsart gewählt wird, so kann es zweckmäßig sein, den Zustand des Reaktionsgemisches durch punktuelle oder fortlaufende Analysen zu untersuchen. Dies bietet den Vorteil, dass gegebenenfalls auch auf Zustandsänderungen im Reaktionsgemisch schnell reagiert werden kann. Darüber hinaus lassen sich so beispielsweise die Reaktionspartner möglichst genau dosieren, um Ausbeuteverluste zu minimieren.

Eine entsprechende Analytik kann beispielsweise durch Probennahme in der Reaktorschlaufe erfolgen. Geeignete Analyseverfahren sind beispielsweise pH-Messung, Messung der Wärmetönung oder Messung der Zusammensetzung des Reaktionsgemisches durch geeignete spektroskopische Verfahren.

Insbesondere im Rahmen der Umsatzkontrolle, Qualitätsaspekten und Sicherheit hat es sich häufig bewährt, den Umsatz im Reaktionsgemisch über die aus dem Reaktionsgemisch abgeführte Wärme zu bestimmen und mit der theoretisch frei werdenden Wärme zu vergleichen.

Die eigentliche Reaktion kann bei geeigneter Wahl des Schlaufenreaktors grundsätzlich in den innerhalb des Schlaufenreaktors angeordneten Rohrsystemen erfolgen. Da die Reaktion jedoch exotherm ist, kann, um Ausbeuteverluste zu vermeiden, auf eine ausreichende Kühlung bzw. auf eine ausreichende Abfuhr der Reaktionswärme geachtet werden. Es hat sich häufig als vorteilhaft erwiesen, wenn die Reaktion innerhalb eines Wärmetauschers, vorzugsweise innerhalb eines Rohrbündelwärmetauschers abläuft. Je nach zu produzierender Produktmenge kann die Kapazität eines entsprechenden Wärmetauschers unterschiedlich gewählt werden. Für großtechnische Verfahren haben sich insbesondere Wärmetauscher mit einem Volumen von 10 bis 40 m³ als besonders geeignet erwiesen. Bei den bevorzugt eingesetzten Rohrbündelwärmetauschern handelt es sich um Wärmetauscher die in einem flüssigkeitsdurchströmten Mantel ein flüssigkeitsdurchströmtes Rohrbündel aufweisen. Je nach Rohrdurchmesser, Packungsdichte, etc. kann der Wärmeübergang zwischen den beiden Flüssigkeiten entsprechend eingestellt werden. Es ist im Rahmen des beschriebenen Verfahrens grundsätzlich möglich, die Reaktion dahingehend zu führen, dass das Reaktionsgemisch durch den Wärmetauscher im Rohrbündel selbst gefahren wird und die Reaktion innerhalb des Rohrbündels stattfindet, wobei die Wärme aus dem Rohrbündel in die Mantelflüssigkeit abgeführt wird.

Es hat sich jedoch ebenfalls als praktikabel und in vielen Fällen als sinnvoll erwiesen, das Reaktionsgemisch durch den Mantel des Wärmetauschers zu führen, während die zur Kühlung eingesetzte Flüssigkeit innerhalb des Rohrbündels zirkuliert. Dabei hat es sich in vielen Fällen als vorteilhaft erwiesen, wenn die Reaktionsmischung im Mantel zur Erzielung einer höheren Verweildauer und einer besseren Durchmischung über Strömungswiderstände, vorzugsweise Umlenkbleche, verteilt wird.

Das Verhältnis von Mantelvolumen zum Volumen des Rohrbündels kann dabei, je nach Auslegung des Reaktors, 10 zu 1 bis 1 zu 10 betragen, vorzugsweise ist das Volumen des Mantels größer als das Volumen des Rohrbündels (bezogen auf den Inhalt der Rohre).

Die Wärmeabfuhr aus dem Reaktor wird mit einem entsprechenden Kühlmittel, beispielsweise mit Wasser, so eingestellt, dass die Reaktionstemperatur innerhalb eines Korridors bei 25 bis 45 °C, insbesondere bei 30 bis 38 °C, insbesondere bei 33 bis 35 °C liegt.

Aus dem Schlaufenreaktor wird kontinuierlich ein Produkt abgeführt. Das Produkt weist eine Temperatur im Rahmen der oben genannten Reaktionstemperaturen, beispielsweise eine Temperatur von 35 °C auf. Das Produkt wird über einen oder mehrere Wärmetauscher, insbesondere über einen oder mehrere Plattenwärmetauscher gekühlt. Dabei kommt beispielsweise eine Solekühlung zum Einsatz. Die Temperatur des Produktes nach Kühlung soll 0 bis 10°C, insbesondere 1 bis 5 °C betragen. Das Produkt wird vorzugsweise in einen Lagerbehälter überführt, der eine Pufferfunktion aufweist. Zusätzlich kann das Produkt im Lagerbehälter beispielsweise durch ständiges Abführen eines Teilstroms aus dem Lagerbehälter zu einem geeigneten Wärmetauscher, beispielsweise zu einem Plattenwärmetauscher, weiter gekühlt werden, bzw. auf einer geeigneten Lagertemperatur gehalten werden. Es ist durchaus möglich, dass in dem Lagerbehälter eine Nachreaktion stattfinden kann.

Die Rückführung des Produktes in den Lagerbehälter, kann grundsätzlich auf beliebige Weise erfolgen. Es hat sich jedoch in einigen Fällen als vorteilhaft herausgestellt, dass das Produkt derart über ein System aus einer oder mehreren Düsen in den Lagerbehälter rückgeführt wird, dass innerhalb des Lagerbehälters eine entsprechende Durchmischung des gelagerten Produktes stattfindet.

Aus dem Lagerbehälter wird weiterhin kontinuierlich Produkt in einen Stabilisierungsbehälter abgeführt. Dort wird das Produkt mit einer geeigneten Säure, beispielsweise mit H₂SO₄, versetzt. Dabei wird der Katalysator deaktiviert und das Reaktionsgemisch auf einen pH-Wert von 1 bis 3, insbesondere 2 eingestellt. Als Säure eignet sich insbesondere Schwefelsäure, beispielsweise Schwefelsäure mit einem Gehalt von 90 bis 105 %, insbesondere von 93 bis 98 % H₂SO₄.

Das stabilisierte Produkt wird dem Stabilisierungsbehälter entnommen und in die Aufreinigungsstufe überführt. Dabei kann ein Teil des entnommenen, stabilisierten Produktes beispielsweise derart in den Stabilisierungsbehälter zurückgeführt werden, dass eine ausreichende Durchmischung des Behälters über ein System aus einer oder mehreren Düsen gewährleistet ist.

Im Rahmen eines weiteren Verfahrenselementes, das im Zusammenhang mit der vorliegenden Erfindung eingesetzt werden kann, wird Acetoncyanhydrin einer destillativen Aufarbeitung unterzogen. Dabei wird das stabilisierte rohe Acetoncyanhydrin über eine entsprechende Kolonne von niedrigsiedenden Bestandteilen befreit. Ein geeignetes Destillationsverfahren kann beispielsweise über nur eine Kolonne geführt werden. Es ist jedoch ebenfalls möglich, im Rahmen einer entsprechenden Aufreinigung von rohem Acetoncyanhydrin eine Kombination von zwei oder mehr Destillationskolonnen auch kombiniert mit einem Fallfilmverdampfer einzusetzen. Weiterhin können zwei oder mehrere Fallfilmverdampfer oder auch zwei oder mehrere Destillationskolonnen miteinander kombiniert werden.

Das rohe Acetoncyanhydrin kommt in der Regel mit einer Temperatur von 0 bis 15 °C, beispielsweise einer Temperatur von 5 bis 10 °C aus der Lagerung zur Destillation.

Grundsätzlich kann das rohe Acetoncyanhydrin direkt in die Kolonne eingeführt werden. Es hat sich jedoch in einigen Fällen bewährt, wenn zunächst das rohe, kühle Acetoncyanhydrin über einen Wärmetauscher einen Teil der Wärme des bereits destillativ gereinigten Produktes übernimmt. Daher wird im Rahmen einer weiteren Ausführungsform des hier beschriebenen Verfahrens das rohe Acetoncyanhydrin über einen Wärmetauscher auf eine Temperatur von 60 bis 80 °C erhitzt.

Die destillative Reinigung des Acetoncyanhydrins erfolgt über eine Destillationskolonne oder eine Rektifikationskolonne mit mehr als 10 Böden oder über eine Kaskade von zwei oder mehr entsprechend geeigneter Destillationskolonnen. Die Beheizung des Kolonnensumpfes erfolgt vorzugsweise mit Dampf. Es hat sich als vorteilhaft herausgestellt, wenn die Sumpftemperatur eine Temperatur von 140 °C nicht übersteigt, gute Ausbeuten und eine gute Reinigung haben sich erzielen lassen, wenn die Sumpftemperatur nicht größer als 130 °C oder nicht höher als 110 °C ist. Die Temperaturangaben beziehen sich dabei auf die Wandtemperatur des Kolonnensumpfes.

Das rohe Acetoncyanhydrin wird im oberen Drittel der Kolonne dem Kolonnenkörper zugeführt. Die Destillation wird vorzugsweise bei verringertem Druck, beispielsweise bei einem Druck von 50 bis 900 mbar, insbesondere 50 bis 250 mbar und mit guten Ergebnissen zwischen 50 bis 150 mbar durchgeführt.

Am Kopf der Kolonne werden gasförmige Verunreinigungen, insbesondere Aceton und Blausäure, entnommen, die abgetrennten gasförmigen Stoffe werden über einen Wärmetauscher oder eine Kaskade von zwei oder mehr Wärmetauschern gekühlt. Hierbei wird vorzugsweise eine Solekühlung mit einer Temperatur von 0 bis 10 °C eingesetzt. Dabei wird den gasförmigen Inhaltsstoffen der Brüden die Gelegenheit gegeben, zu kondensieren. Die erste Kondensationsstufe kann beispielsweise bei Normaldruck stattfinden. Es ist jedoch ebenso möglich und hat sich in einigen Fällen als vorteilhaft erwiesen, wenn diese erste Kondensationsstufe unter verringertem Druck, vorzugsweise bei dem Druck, der im Rahmen der Destillation vorherrscht, erfolgt. Das Kondensat wird in einen gekühlten Auffangbehälter weitergeleitet und dort bei einer Temperatur von 0 bis 15 °C, insbesondere bei 5 bis 10 °C gesammelt.

Die im Rahmen des ersten Kondensationsschrittes nicht kondensierenden gasförmigen Verbindungen werden über eine Vakuumpumpe aus dem Unterdruckraum entfernt. Hierbei ist grundsätzlich eine beliebige Vakuumpumpe einsetzbar. Es hat sich jedoch in vielen Fällen als vorteilhaft erwiesen, wenn eine Vakuumpumpe eingesetzt wird, die aufgrund ihrer Bauweise nicht zum Eintrag flüssiger Verunreinigungen in den Gasstrom führt. Vorzugsweise werden hier daher beispielsweise trocken laufende Vakuumpumpen eingesetzt.

Der auf der Druckseite der Pumpe entweichende Gasstrom wird über einen weiteren Wärmetauscher geführt, der vorzugsweise mit Sole bei einer Temperatur von 0 bis 15 °C gekühlt wird. Hierbei kondensierende Inhaltsstoffe werden ebenfalls in dem Sammelbehälter gesammelt, der bereits die unter Vakuumbedingungen gewonnenen Kondensate auffängt. Die auf der Druckseite der Vakuumpumpe durchgeführte Kondensation kann beispielsweise durch einen Wärmetauscher, jedoch auch mit einer Kaskade von zwei oder mehr seriell parallel angeordneten Wärmetauschern erfolgen. Nach diesem Kondensationsschritt verbleibende gasförmigen Stoffen werden abgeführt und einer beliebigen weiteren Verwertung, beispielsweise einer thermischen Verwertung, zugeführt.

Die gesammelten Kondensate können ebenfalls beliebig weiterverwertet werden. Es hat sich jedoch als unter ökonomischen Gesichtspunkten äußerst vorteilhaft erwiesen, die Kondensate in die Reaktion zur Herstellung von Acetoncyanhydrin zurückzuführen. Dies erfolgt vorzugsweise an einer oder mehreren Stellen, die Zugang zum Schlaufenreaktor ermöglichen. Die Kondensate können grundsätzlich eine beliebige Zusammensetzung aufweisen, sofern sie die Herstellung des Acetoncyanhydrins nicht stören. In vielen Fällen wird die überwiegende Menge des Kondensates jedoch aus Aceton und Blausäure bestehen, beispielsweise in einem molaren Verhältnis von 2:1 bis 1:2, häufig in einem Verhältnis von 1:1.

Das aus dem Sumpf der Destillationskolonne gewonnene Acetoncyanhydrin wird zunächst über einen ersten Wärmetauscher durch das zugeführte, kalte rohe Acetoncyanhydrin auf eine Temperatur von 40 bis 80 °C abgekühlt. Anschließend wird das Acetoncyanhydrin über einen mindestens einen weiteren Wärmetauscher auf eine Temperatur von 30 bis 35 °C gekühlt und ggf. zwischengelagert.

Insgesamt hat es sich in einigen Fällen als vorteilhaft herausgestellt, wenn das Acetoncyanhydrin in einer Rektifikationskolonne mindestens von Verunreinigungen mit einem Siedepunkt von mehr als -5 °C und weniger als 100 °C, beispielsweise mehr als 0 °C und weniger als 90 °C, befreit wird und diese Verunreinigungen in die Reaktion zur Herstellung von Acetoncyanhydrin zurückgeführt werden. Die entsprechende Verfahrensvariante wird vorteilhafterweise mit Hilfe einer Vorrichtung durchgeführt, die eine Rektifikationskolonne zum Entfernen von Bestandteilen mit einem Siedepunkt von mehr als - 5 °C und weniger als 100 °C aus den hergestellten Acetoncyanhydrin aufweist und die Rektifikationskolonne derart fluidleitend mit dem Anlagenelement zur Herstellung von Acetoncyanhydrin verbunden ist, dass die entfernten Bestandteile in die Reaktion zur Herstellung von Acetoncyanhydrin zurückgeführt werden können.

### Schritt b) Umsetzung von Acetoncyanhydrin zu Methacrylamid

Im Rahmen eines weiteren Verfahrensschritts wird das im ersten Schritt hergestellte Acetoncyanhydrin einer Hydrolyse unterzogen. Die Umsetzung erfolgt in einer dem Fachmann bekannten Weise durch eine Reaktion zwischen konzentrierter Schwefelsäure und Acetoncyanhydrin.

Die Umsetzung kann auch hier wieder im Batchverfahren oder im kontinuierlichen Verfahren durchgeführt werden. Letzteres hat sich in vielen Fällen als vorteilhaft erwiesen. Sofern die Umsetzung im Rahmen eines kontinuierlichen Verfahrens durchgeführt wird, hat sich der Einsatz von Schlaufenreaktoren bewährt. Die Umsetzung kann beispielsweise in nur einem Schlaufenreaktor erfolgen. Es kann jedoch vorteilhaft sein, wenn die Umsetzung in einer Kaskade von zwei oder mehreren Schlaufenreaktoren durchgeführt wird.

Ein geeigneter Schlaufenreaktor weist im Rahmen des beschriebenen Verfahrens eine oder mehrere Zufuhrstellen für Acetoncyanhydrin, eine oder mehrere Zufuhrstellen für konzentrierte Schwefelsäure, einen oder mehrere Gasabscheider, einen oder mehrere Wärmetauscher und eine oder mehrere Mischer auf.

Die Hydrolyse von Acetoncyanhydrin mit Schwefelsäure zu Methacrylamid ist, wie bereits beschrieben, exotherm. Die im Rahmen der Reaktion anfallende Reaktionswärme kann dem System jedoch vorteilhafterweise zumindest derart weitgehend entzogen werden, dass eine Ausbeutemaximierung erzielt werden kann, da mit zunehmender Temperatur bei der Umsetzung die Ausbeute sinkt. Es ist dabei grundsätzlich möglich mit entsprechenden Wärmetauschern eine schnelle und umfassende Abfuhr der Reaktionswärme zu erzielen. Es kann jedoch vorteilhaft sein, das Gemisch nicht zu sehr zu kühlen, da für einen entsprechenden Austausch an den Wärmetauschern ein ausreichender Wärmeübergang erforderlich ist. Da mit sinkender Temperatur die Viskosität des Gemischs ansteigt, kann bei zu starker Kühlung die Zirkulation im Schlaufenreaktor erschwert werden. Dabei kann gegebenenfalls eine ausreichende Abfuhr der Reaktionsenergie aus dem System nicht mehr gewährleistet sein.

Darüber hinaus können zu geringe Temperaturen im Reaktionsgemisch zu einer Kristallisation von Inhaltsstoffen des Reaktionsgemischs an den Wärmetauschern führen. Dadurch kann der Wärmeübergang weiter verschlechtert werden, was gegebenenfalls einen Ausbeuterückgang nach sich zieht. Darüber hinaus kann eine zu starke Kühlung zurfolge haben, dass der Schlaufenreaktor nicht mit den optimalen Mengen an Reaktanten beschickt werden kann, so dass gegebenenfalls die Effizienz des Verfahrens leiden kann.

Im Rahmen einer weiteren Ausgestaltung der Erfindung kann aus einem Strom von Acetoncyanhydrin ein Teil, beispielsweise zwei Drittel bis drei Viertel, des Volumenstroms in einen ersten Schlaufenreaktor eingeführt. Dabei kann ein solcher erster Schlaufenreaktor einen oder mehrere Wärmetauscher, eine oder mehrere Pumpen, einen oder mehrere Mischelemente und einen oder mehrere Gasabscheider aufweisen. Die den ersten Schlaufenreaktor durchlaufenden Umwälzströme liegen beispielsweise im Bereich von 100 bis 450 m³/h, bevorzugt in einem Bereich von 200 bis 400 m³/h und darüber hinaus bevorzugt in einem Bereich von 250 bis 350 m³/h. In einen auf den ersten Schlaufenreaktor folgenden mindestens einen weiteren Schlaufenreaktor liegen die Umwälzströme vorzugsweise in einem Bereich von 40 bis 450 m³/h, bevorzugt in einem Bereich von 50 bis 400 m³/h und darüber hinaus bevorzugt in einem Bereich von 60 bis 350 m³/h. Weiterhin sind als Temperaturdifferenz über den Wärmetauscher 1 bis 10 °C bevorzugt, wobei 2 bis 7 °C besonders bevorzugt sind.

Die Zufuhr des Acetoncyanhydrin kann grundsätzlich an beliebiger Stelle in den Schlaufenreaktor erfolgen. Es hat sich jedoch als vorteilhaft erwiesen, wenn die Zufuhr in ein Mischelement, beispielsweise in einen Mischer mit beweglichen Teilen oder einen Statikmischer, erfolgt. Die Zufuhr der Schwefelsäure erfolgt vorteilhafter Weise vor der Acetoncyanhydrin-Zugabe. Ansonsten ist es jedoch ebenfalls möglich, die Schwefelsäure an beliebiger Stelle in den Schlaufenreaktor einzuführen.

Das Verhältnis der Reaktanten im Schlaufenreaktor wird beispielsweise so gesteuert, dass ein Überschuss an Schwefelsäure vorliegt. Der Überschuss an Schwefelsäure kann, bezüglich des molaren Verhältnisses der Inhaltsstoffe, im ersten Schlaufenreaktor 1,8:1 bis 3:1 und im letzten Schlaufenreaktor 1,3:1 bis 2:1 betragen.

In einigen Fällen hat es sich als vorteilhaft erwiesen, mit einem derartigen Überschuss an Schwefelsäure die Reaktion im Schlaufenreaktor zu betreiben. Die Schwefelsäure kann hier beispielsweise als Lösemittel dienen und die Viskosität des Reaktionsgemischs niedrig halten, wodurch eine höhere Abfuhr von Reaktionswärme und eine niedrigere Temperatur des Reaktionsgemischs gewährleistet werden kann. Dies kann deutliche Ausbeutevorteile mit sich bringen. Die Temperatur im Reaktionsgemisch beträgt 90 bis 120 °C, beispielsweise 95 bis 115 °C.

Die Wärmeabfuhr kann durch einen oder mehrere Wärmetauscher im Schlaufenreaktor gewährleistet werden. Es hat sich dabei oft als vorteilhaft erwiesen, wenn die Wärmetauscher über eine geeignete Sensorik zum Einstellen der Kühlleistung verfügen, um eine zu starke Kühlung des Reaktionsgemischs aus den oben genannten Gründen zu verhindern. So kann es beispielsweise vorteilhaft sein, den Wärmeübergang im Wärmetauscher oder in den Wärmetauschern punktförmig oder kontinuierlich zu messen und daran die Kühlleistung der Wärmetauscher anzupassen. Dies kann beispielsweise über das Kühlmittel selbst geschehen. Es ist auch ebenso möglich durch entsprechende Variation der Zugabe der Reaktionspartner und durch die Erzeugung von mehr Reaktionswärme eine entsprechende Erhitzung des Reaktionsgemisches zu erreichen. Auch eine Kombination von beiden Möglichkeiten ist denkbar. Der Schlaufenreaktor sollte darüber hinaus über mindestens einen Gasabscheider verfügen. Über den Gasabscheider wird einerseits dem Schlaufenreaktor kontinuierlich gebildetes Produkt entnommen. Andererseits lassen sich im Rahmen der Reaktion gebildete Gase so dem Reaktionsraum entziehen. Als Gas bildet sich hauptsächlich Kohlenmonoxid. Das aus dem Schlaufenreaktor entnommene Produkt wird vorzugsweise in einen zweiten Schlaufenreaktor überführt. In diesen zweiten Schlaufenreaktor wird das Schwefelsäure und Methacrylsäureamid beinhaltende Reaktionsgemisch wie es durch die Reaktion im ersten Schlaufenreaktor erhalten wurde, mit dem verbleibenden Teilstrom an Acetoncyanhydrin umgesetzt. Hierbei reagiert der Überschuss an Schwefelsäure aus dem ersten Schlaufenreaktor, oder zumindest ein Teil der überschüssigen Schwefelsäure, mit dem Acetoncyanhydrin unter weiterer Bildung von Methacrylamid. Die Durchführung der Reaktion in zwei oder mehr Schlaufenreaktoren weist den Vorteil auf, dass aufgrund des Schwefelsäureüberschusses im ersten Schlaufenreaktor die Pumpbarkeit des Reaktionsgemischs und damit der Wärmeübergang und letztendlich die Ausbeute verbessert werden. Im zweiten Schlaufenreaktor sind wiederum mindestens ein Mischelement, mindestens ein Wärmetauscher und mindestens ein Gasabscheider angeordnet. Die Reaktionstemperatur im zweiten Schlaufenreaktor beträgt ebenfalls 90 bis 120 °C.

Das Problem der Pumpbarkeit des Reaktionsgemischs, des Wärmeübergangs und einer möglichst geringen Reaktionstemperatur stellt sich in jedem weiteren Schlaufenreaktor genauso wie im ersten. Deshalb verfügt vorteilhafter Weise auch der zweite Schlaufenreaktor über einen Wärmetauscher, dessen Kühlleistung durch entsprechende Sensorik geregelt werden kann.

Die Zufuhr des Acetoncyanhydrins erfolgt wiederum in einem geeigneten Mischelement, vorzugsweise in einen Statikmischer.

Aus dem Gasabscheider des zweiten Schlaufenreaktors wird das Produkt entnommen und zur Vervollständigung der Umsetzung und zur Bildung des Methacrylamids auf eine Temperatur von 140 bis 180 °C erhitzt.

Die Erhitzung wird vorzugsweise derart durchgeführt, dass die Maximaltemperatur nur für einen möglichst kurzen Zeitraum, beispielsweise für eine Zeit von 1-30 min, insbesondere für eine Zeit von 2-8 bevorzugt 3-5 min erreicht wird. Dies kann grundsätzlich in beliebigen Apparaturen zur Erzielung einer derartigen Temperatur für einen derart kurzen Zeitraum erfolgen. Beispielsweise kann die Energiezufuhr auf konventionellem Wege durch elektrische Energie oder durch Dampf erfolgen. Es ist jedoch ebenso möglich, die Energie durch elektromagnetische Strahlung, beispielsweise durch Mikrowellen zuzuführen.

Es hat sich in verschiedenen Fällen als vorteilhaft erwiesen, wenn der Erhitzungsschritt in einem Wärmetauscher mit zwei oder mehrstufiger Anordnung von Rohrwendeln, die vorzugsweise in einer mindestens doppelten, gegenläufigen Anordnung vorliegen können, erfolgt. Dabei wird das Reaktionsgemisch schnell auf eine Temperatur von 140 bis 180 °C erhitzt.

Der Wärmetauscher kann beispielsweise mit einem oder mehreren Gasabscheidern kombiniert werden. So ist es beispielsweise möglich, das Reaktionsgemisch nach Verlassen der ersten Rohrwendel im Wärmetauscher durch einen Gasabscheider zu führen. Dabei können beispielsweise während der Reaktion entstehende, gasförmige Komponenten aus dem Reaktionsgemisch abgetrennt werden. Es ist ebenso möglich, das Reaktionsgemisch nach Verlassen der zweiten Wendel mit einem Gasabscheider zu behandeln. Es kann sich darüber hinaus als vorteilhaft erweisen, an beiden Stellen, sowohl nach Verlassen der ersten als auch nach Verlassen der zweiten Rohrwendel das Reaktionsgemisch mit einem Gasabscheider zu behandeln.

Die so erhältliche Amidlösung weist in der Regel eine Temperatur von mehr als 100 °C, üblicherweise eine Temperatur von 140 bis 180 °C auf.

Die im Rahmen der Amidierung anfallenden gasförmigen Verbindungen können grundsätzlich in beliebiger Weise entsorgt oder einer Weiterverarbeitung zugeführt werden. Es kann in einigen Fällen jedoch vorteilhaft sein, wenn die entsprechenden Gase in einer Transportleitung dergestalt zusammengeführt werden, dass sie entweder kontinuierlich oder bei Bedarf ggf. mit Druck, beispielsweise mit Dampfdruck, beaufschlagt und so weitertransportiert werden können.

Im Rahmen einer weiteren Ausführungsform der Erfindung hat es sich in einigen Fällen als vorteilhaft herausgestellt, wenn im Rahmen der Herstellung von Methacrylamid anfallende gasförmige Produkte im Rahmen des Weitertransportes in das Reaktionsgemisch der nachfolgend geschilderten Veresterung eingeleitet werden. Es kann eine solche Einleitung dabei grundsätzlich an einer beliebigen Stelle der Veresterung erfolgen. Oftmals ist es jedoch vorteilhaft, insbesondere dann, wenn eine Veresterung in mehreren Kesseln erfolgt, die anfallenden gasförmigen Produkte in das Reaktionsgemisch der Veresterung einzuleiten, das sich in einem ersten Kessel befindet. Die Einleitung der anfallenden gasförmigen Produkte kann beispielsweise derart ausgestaltet werden, dass die mit Dampf beaufschlagten Gase so in einen Kessel eingeleitet werden, dass sie für eine zumindest lokale Durchmischung des Kesselinhalts sorgen oder für eine Erwärmung des Kesselinhalts oder für eine im wesentlichen konstante Temperatur des Kesselinhalts oder für eine Kombination aus zwei der genannten Elemente sorgen.

### Schritt c) Veresterung von Methacrylamid in Gegenwart von Alkoholen zu Methacrylsäureestern

Ein weiterer erfindungsgemäßer Schritt ist die Alkoholyse von Methacrylamid zu den entsprechenden Methacrylsäureestern. Diese Reaktion kann in einem oder mehreren beheizten, beispielsweise durch Dampf beheizten Kesseln, durchgeführt werden. Es hat sich in vielen Fällen als vorteilhaft erwiesen, wenn die Veresterung in mindestens zwei aufeinander folgenden Kesseln, beispielsweise jedoch auch in drei oder vier oder mehr aufeinander folgenden Kesseln durchgeführt wird. Dabei wird eine Lösung von Methacrylamid in den Kessel bzw. in den ersten Kessel einer zwei oder mehr Kessel umfassenden Kaskade von Kesseln eingeführt.

Es ist häufig bevorzugt, eine entsprechende Veresterungsreaktion mit einer Kaskade von zwei oder mehr Kesseln durchzuführen. Im Folgenden soll daher ausschließlich auf diese Variante Bezug genommen werden.

Im Rahmen der hier beschriebenen Erfindung kann beispielsweise eine Amidlösung, wie sie aus der hier beschriebenen Amidierungsreaktion erhältlich ist, in einen ersten Kessel eingespeist werden. Der Kessel wird beispielsweise mit Dampf beheizt. Die zugeführte Amidlösung weist in der Regel eine erhöhte Temperatur auf, beispielsweise eine Temperatur von 100 bis 180 °C, im Wesentlichen entsprechend der Ablauftemperatur der Amidlösung aus der oben vorgestellten Amidierungsreaktion. Den Kesseln wird weiterhin ein Alkanol zugeführt, das zur Veresterung eingesetzt werden kann.

Grundsätzlich eignen sich hier beliebige Alkanole mit 1 bis 4 Kohlenstoffatomen, die linear oder verzweigt, gesättigt oder ungesättigt sein können, wobei Methanol besonders bevorzugt ist. Ebenso können diese Alkanole zusammen mit Methacrylsäureestern eingesetzt werden, was insbesondere bei Umesterungen der Fall ist.

Der Kessel wird weiterhin mit Wasser beschickt, so dass insgesamt eine Wasserkonzentration im Kessel von 13 bis 26 Gew.-%, insbesondere 18 bis 20 Gew.-% herrscht.

Die Menge an Amidlösung und an Alkanol wird derart geregelt, dass ein Gesamtmolverhältnis von Amid zu Alkanol von 1:1,4 bis 1:1,6 herrscht. Das Alkanol kann auf die Kesselkaskade derart verteilt werden, dass im ersten Reaktor das Molverhältnis 1:1,1 bis 1:1,4 beträgt und in den folgenden Reaktionsstufen bezogen auf den Gesamtamidstrom Molverhältnisse von 1:0,05 bis 1:0,3 eingestellt werden. Das in die Veresterung zugeführte Alkanol kann sich aus "Frischalkanol" sowie Alkanol aus Recyclingströmen der Aufarbeitungsstufen und bei Bedarf auch aus Recyclingströmen der Downstreamprozesse des Produktionsverbundes zusammensetzen.

Die Beschickung des ersten Kessels mit Wasser kann grundsätzlich dahingehend erfolgen, dass Wasser aus einer beliebigen Quelle dem Kessel zugeführt wird, sofern dieses Wasser keine Inhaltsstoffe aufweist, die die Veresterungsreaktion oder die nachfolgenden Prozessstufen nachteilig beeinflussen könnten. Beispielsweise kann dem Kessel VE-Wasser oder Brunnenwasser zugeführt werden. Es ist jedoch ebenfalls möglich, dem Kessel ein Gemisch aus Wasser und organischen Verbindungen zuzuführen, wie sie beispielsweise bei der Reinigung von Methacrylsäure oder Methacrylsäureestern anfallen. Im Rahmen einer bevorzugten Ausführungsform des hier vorgestellten Verfahrens werden die Kessel zumindest anteilig mit einem Gemisch aus Wasser und solchen organischen Verbindungen beschickt.

Wenn eine Kaskade von zwei oder mehr Kesseln im Rahmen der Veresterungsreaktion eingesetzt wird, so können die entstandenen gasförmigen Stoffe, insbesondere der Methacrylsäureester, grundsätzlich aus jedem Kessel einzeln abgezogen und einer Reinigung zugeführt werden. Es hat sich jedoch in manchen Fällen als vorteilhaft erwiesen, wenn bei einer Kaskade von zwei oder mehr Kesseln die gasförmigen Produkte aus dem ersten Kessel zunächst in den zweiten Reaktionskessel eingespeist werden, ohne dass die gasförmigen Verbindungen aus dem ersten Kessel direkt einer Reinigung zugeführt werden. Diese Vorgehensweise bietet den Vorteil, dass der im ersten Kessel häufig starken Schaumentwicklung nicht durch aufwendige, apparative Entschäumung begegnet werden muss. Im Falle einer Kaskadierung der gasförmigen Stoffe aus dem ersten Kessel in den zweiten Kessel tritt der im ersten Kessel gebildete und ggf. mitgerissene Schaum einfach in den Reaktionsraum des zweiten Kessels mit ein. Da dort in der Regel die Schaumbildung deutlich geringer ist, muss so nicht apparativ entschäumt werden.

Der nach einem ersten Kessel angeordnete zweite Kessel nimmt nun einerseits den Überlauf des ersten Kessels auf, andererseits wird er mit den gasförmigen im ersten Kessel gebildeten oder im ersten Kessel vorhandenen Stoffen gespeist. Der zweite Kessel und die evt. folgenden werden ebenfalls mit Methanol beschickt. Hierbei ist es bevorzugt, dass die Methanolmenge von Kessel zu Kessel um mindestens 10 %, jeweils auf den vorhergehenden Kessel bezogen, abnimmt. Die Wasserkonzentration im zweiten Kessel sowie in den weiteren Kesseln kann sich von der des ersten Kessels unterscheiden, oftmals sind die Konzentrationsunterschied jedoch gering.

Die im zweiten Kessel entstehenden Brüden werden aus dem Kessel abgeführt und in den Sumpf einer Destillationskolonne eingeleitet.

Wenn die Veresterung mit einer Kaskade von drei oder mehr Kesseln durchgeführt wird, so wird jeweils der Überlauf des zweiten Kessels in einen dritten Kessel überführt sowie der Überlauf des dritten Kessels ggf. in einen vierten Kessel überführt. Die weiteren Kessel werden ebenfalls dampfbeheizt. Vorzugsweise wird die Temperatur in den Kesseln 3 und ggf. 4 auf 120 °C bis 140 °C eingestellt.

Die aus den Kesseln entweichenden Brüden werden in eine Destillationskolonne eingeleitet, wobei diese vorzugsweise im Unterbereich der Destillationskolonne erfolgt. Die Brüden umfassen ein azeotropes Gemisch aus Träger-Dampf, Methacrylsäureester und Alkanol und weisen je nach eingesetztem Alkanol eine Temperatur von 60 bis 120 °C, beispielsweise 70 bis 90 °C bei Einsatz von Methanol auf. In der Destillationskolonne wird der Methacrylsäureester gasförmig von den bei höheren Temperaturen siedenden Brüdenbestandteilen abgetrennt. Die hochsiedenden Anteile (hauptsächlich Methacrylamid, Hydroxyisobuttersäureester und Wasser) werden in den ersten Reaktionskessel zurückgeführt. Der gebildete Methacrylsäureester wird am Kolonnenkopf abgezogen und über einen Wärmetauscher oder eine Kaskade von zwei oder mehr Wärmetauschern abgekühlt. Es hat sich in einigen Fällen bewährt, wenn die Kühlung des Methacrylsäureesters über mindestens zwei Wärmetauscher erfolgt, wobei ein erster Wärmetauscher mit Wasser die Kondensation und eine Kühlung auf eine Temperatur von 60 bis 30 °C durchführt, während ein zweiter, solegekühlter Wärmetauscher eine Abkühlung auf 5 bis 15 °C vornimmt. Von dem wassergekühlten Kondensat kann ein Teilstrom als Rücklauf auf die Kolonnen zur Konzentrationssteuerung der Kolonne gegeben werden. Es ist jedoch ebenso möglich, den gebildeten Methacrylsäureester über eine Kaskade von mehr als zwei Wärmetauschern zu kühlen. Hierbei ist es beispielsweise möglich, zunächst eine Kühlung über zwei hintereinander geschaltete, wassergekühlte Wärmetauscher vorzunehmen und anschließend über einen entsprechenden solegekühlten Wärmetauscher eine weitere Abkühlung zu erzielen.

So kann beispielsweise im Rahmen des hier vorgestellten Verfahrens der gebildete Methacrylsäureester in gasförmigem Zustand über einen ersten Wärmetauscher mit Wasserkühlung gekühlt werden. Sowohl kondensierte als auch nicht kondensierte Stoffe werden anschließend in einen zweiten Wärmetauscher weitergeleitet, wo eine weitere Kondensation über Wasserkühlung stattfindet. An dieser Stelle können nun beispielsweise gasförmige Stoffe in einen separaten, mit Sole gekühlten Wärmetauscher überführt werden. Das Kondensat in diesem solegekühlten Wärmetauscher wird anschließend in den Destillatstrom gegeben, während die verbleibenden gasförmigen Stoffe weiter verwertet werden können oder einer Entsorgung zugeführt werden. Das Methacrylsäureester-Kondensat aus dem zweiten wassergekühlten Wärmetauscher wird nun in einem mit Wasser oder mit Sole gekühlten Wärmetauscher auf eine Temperatur von weniger als 15 °C, vorzugsweise 8 bis 12 °C gekühlt. Dieser Kühlschritt kann dazu führen, dass der gebildete Methacrylsäureester einen deutlich niedrigeren Gehalt an Ameisensäure aufweist, als dies ohne den entsprechenden Kühlungsschritt der Fall wäre. Das abgekühlte Kondensat wird anschließend in einen Phasentrenner überführt. Hier wird die organische Phase (Methacrylsäureester) von der wässrigen Phase getrennt. Die wässrige Phase, die neben Wasser noch einen Gehalt an organischen Verbindungen, insbesondere Alkanol, aus dem Destillationsschritt aufweisen kann, kann grundsätzlich beliebig weiterverwendet werden. Wie jedoch bereits oben beschrieben, kann es bevorzugt sein, dieses Gemisch aus Wasser und organischen Verbindungen wieder in den Veresterungsprozess zurückzuführen, indem eine Einspeisung in den ersten Reaktionskessel erfolgt.

Die abgetrennte organische Phase wird in einen Wäscher eingespeist. Dort wird der Methacrylsäureester mit demineralisiertem Wasser gewaschen. Die abgeschiedene wässrige Phase, die ein Gemisch aus Wasser und organischen Verbindungen, insbesondere Alkanol, enthält, kann wiederum grundsätzlich beliebig weiterverwendet werden. Es ist jedoch unter ökonomischen Gesichtspunkten vorteilhaft, diese wässrige Phase wieder in den Veresterungsschritt rückzuführen, indem sie beispielsweise in den ersten Kessel eingespeist wird.

Da Methacrylsäureester eine starke Neigung zur Polymerisation aufweisen, ist es in vielen Fällen vorteilhaft, wenn im Rahmen der Alkoholyse des Methacrylamids dafür Sorge getragen wird, dass eine derartige Polymerisation verhindert wird.

In Anlagen zur Herstellung von Methacrylsäure oder Methacrylsäureestern findet Polymerisation oft dann statt, wenn die Stoffströme eine zu geringe Strömungsgeschwindigkeit aufweisen, so dass sich lokal Ruhezonen ausbilden können, in denen ein über längere Zeit andauernder Kontakt zwischen den polymerisierbaren Bestandteilen und einem Polymerisationsinitiator einstellen kann, der in Folge dann zur Polymerisation führen kann.

Um ein entsprechendes Polymerisationsverhalten zu vermeiden, kann es vorteilhaft sein, eine Optimierung des Stoffflusses dahingehend durchzuführen, dass einerseits die Strömungsgeschwindigkeit der Stoffströme an möglichst allen Stellen im System so hoch ist, dass die Zahl der Ruhezonen minimiert wird. Darüber hinaus kann es vorteilhaft sein, die Stoffströme derart mit geeigneten Stabilisatoren zu versetzen, dass eine Polymerisation weitgehend unterdrückt wird.

Zu diesem Zweck können im Rahmen des hier dargestellten Verfahrens grundsätzlich die Stoffströme derart mit Stabilisatoren versetzt werden, dass möglichst wenig Polymerisation im System selbst stattfindet. Hierzu wird insbesondere der Teil der Anlage mit entsprechenden Stabilisatoren versorgt, in dem die Methacrylsäureester während oder nach der Destillation in hoher Konzentration vorliegen.

So hat es sich beispielsweise als sinnvoll erwiesen, am Kopf der Destillationskolonne dem dort abgezogenen Strom an Methacrylsäureester einen Stabilisator zuzuführen. Weiterhin hat es sich als vorteilhaft erwiesen, diejenigen Anlagenteile mit einer Lösung von Stabilisator in Methacrylsäureester zu spülen, in denen Methacrylsäure oder Methacrylsäureester mit einer Temperatur von mehr als 20 °C, vorzugsweise mit einer Temperatur im Bereich von 20 bis 120 °C zirkuliert. So wird beispielsweise ein Teil des in den Wärmetauschern anfallenden Kondensates zusammen mit einem geeigneten Stabilisator derart in den Kopf der Destillationskolonne zurückgeführt, dass dort der Kolonnenkopf an seiner Innenseite ständig mit stabilisiertem Methacrylsäureester oder stabilisierter Methacrylsäure besprüht wird. Dies geschieht vorzugsweise derart, dass sich im Kolonnenkopf keine Ruhezonen ausbilden können, an denen eine Polymerisation von Methacrylsäure oder Methacrylsäureester zu befürchten ist. Die Wärmetauscher selbst können entsprechend ebenfalls mit einer stabilisierten Lösung von Methacrylsäure oder Methacrylsäureester derart beaufschlagt werden, dass auch hier keine Ruhezonen zur Ausbildung gelangen können.

Es hat sich weiterhin als vorteilhaft im Rahmen des hier vorgestellten Verfahrens gezeigt, wenn beispielsweise die Kohlenmonoxid enthaltenden Abgase aus vorangehenden Prozessen, insbesondere aus dem Amidierungsschritt, zusammen mit Dampf durch die Veresterungsanlage geleitet werden. Auf diese Weise erfolgt eine nochmalige Vereinigung des Gasgemisches von Verbindungen, die als Feststoff oder als Flüssigkeit abgetrennt werden können. Zum anderen werden diese an einer zentralen Stelle gesammelt und können der weiteren Verwertung oder Entsorgung zugeführt werden.

Das im Rahmen der Veresterung und der anschließenden Vorreinigung erhaltene MMA bzw. der erhaltene Methacrylsäureester werden anschließend einer weiteren Behandlung zugeführt. Aus der Veresterung resultiert als verbleibender Reststoff verdünnte Schwefelsäure, die ebenfalls einer weiteren Verwertung zugeführt werden kann.

Optional kann im Rahmen des Verfahrens der vorliegenden Erfindung auch ein Verfahren zur Vorreinigung von Methacrylsäureester eingesetzt werden, wie dieses in den nachfolgenden Verfahrensschritten beschrieben wird. Vorteilhafterweise umfasst die Reinigung zwei Stufen. In einer ersten Vorreinigung werden die niedrigsiedenden Bestandteile des Produktes entfernt. Hierzu wird roher Methacrylsäureester zunächst in eine Destillationskolonne überführt, in welcher die niedrigsiedenden Bestandteile und Wasser abgetrennt werden können. Hierzu wird der rohe Methacrylsäureester einer Destillationskolonne zugeführt, wobei die Zugabe in der oberen Hälfte der Kolonne durchgeführt wird. Der Kolonnensumpf wird mit Dampf beispielsweise derart beheizt, dass eine Wandtemperatur von 50 bis 120 °C erreicht wird. Die Reinigung wird unter Vakuum durchgeführt. Der Druck innerhalb der Kolonne beträgt im Fall des Esters vorzugsweise 100 bis 600 mbar.

Am Kolonnenkopf werden die niedrigsiedenden Bestandteile abgenommen. Insbesondere können dieses beispielsweise Ether, Aceton und Methylformiat sein. Die Brüden werden anschließend über einen oder mehrere Wärmetauscher kondensiert. Dabei hat es sich beispielsweise in einigen Fällen bewährt, zunächst eine Kondensation über zwei in Serie geschaltete, mit Wasser gekühlte Wärmetauscher durchzuführen. Es ist jedoch ebenso möglich, an dieser Stelle auch nur einen Wärmetauscher einzusetzen. Die Wärmetauscher werden vorzugsweise zur Erhöhung der Flussgeschwindigkeit und um die Ausbildung stationärer Phasen zu verhindern, in senkrechtem Zustand betrieben. Dem wassergekühlten Wärmetauscher oder den wassergekühlten Wärmetauschern nachgeschaltet, kann ein solegekühlter Wärmetauscher sein, es ist jedoch auch möglich, eine Kaskade von zwei oder mehr solegekühlten Wärmetauschern nachzuschalten. In der Kaskade von Wärmetauschern werden die Brüden kondensiert, mit Stabilisator versehen und beispielsweise einem Phasentrenner zugeführt. Da die Brüden auch Wasser enthalten können, wird eine evtl. anfallende wässrige Phase entsorgt oder einer weiteren Verwertung zugeführt. Als weitere Verwertung bietet sich beispielsweise die Rückführung in eine Veresterungsreaktion, beispielsweise in eine Veresterungsreaktion wie sie oben beschrieben wurde, an. In diesem Fall wird die wässrige Phase vorzugsweise in den ersten Veresterungskessel rückgeführt.

Die abgetrennte organische Phase wird als Rückfluss in den Kolonnenkopf eingespeist. Ein Teil der organischen Phase kann wiederum zum Besprühen der Wärmetauscherköpfe und des Kolonnenkopfes eingesetzt werden. Da es sich bei der abgetrennten organischen Phase um eine Phase handelt, die mit Stabilisator versetzt ist, lässt sich so wirksam einerseits die Ausbildung von Ruhezonen verhindern. Andererseits bewirkt die Gegenwart des Stabilisators eine weitere Unterbindung der Polymerisationsneigung der abgetrennten Brüden.

Der aus den Wärmetauschern erhaltene Kondensatstrom wird darüber hinaus vorzugsweise derart mit demineralisiertem Wasser versetzt, dass im Phasentrenner eine ausreichende Abscheidwirkung erzielt werden kann.

Die nach der Kondensation in der Wärmetauscherkaskade verbleibenden, gasförmigen Verbindungen können, vorzugsweise mittels Dampfstrahler als Unterdruckerzeuger, nochmals über einen oder mehrere weitere Wärmetauscher einer Kondensation unterzogen werden. Es hat sich dabei unter ökonomischen Gesichtspunkten als vorteilhaft herausgestellt, wenn im Rahmen einer solchen Nachkondensation nicht nur die gasförmigen Stoffe aus der Vorreinigung kondensiert werden. So ist es beispielsweise möglich, einer derartigen Nachkondensation weitere gasförmige Stoffe zuzuführen, wie sie sich aus der Hauptreinigung von Methacrylsäureestern ergeben. Der Vorteil einer derartigen Verfahrensweise liegt beispielsweise darin, dass so ein Anteil an Methacrylsäureester, der im Rahmen der Hauptreinigungsstufe nicht kondensiert wurde, im Rahmen der Vorreinigung nochmals über den Phasenabscheider in die Reinigungskolonne überführt werden kann. So wird beispielsweise gewährleistet, dass eine Ausbeutemaximierung stattfinden kann, und möglichst geringe Verluste an Methacrylsäureester auftreten. Außerdem kann durch die geeignete Wahl der Auslegung und des Betriebs dieser weiteren Wärmetauscher die Zusammensetzung des diese Wärmetauscher verlassenden Abgases, insbesondere der Gehalt an Leichtsiedern, eingestellt werden.

Aufgrund der Zuführung von Wasser im Rahmen der Vorreinigung des Methacrylsäureesters kann der Wassergehalt in der Veresterung und die Konzentration an niedrigsiedenden Bestandteilen im Roh-Methacrylsäureester insgesamt kontinuierlich ansteigen. Um dies zu vermeiden, kann es vorteilhaft sein, einen Teil des dem System zugeführten Wassers, vorzugsweise kontinuierlich, aus dem System auszuschleusen. Dieses Ausschleusen kann grundsätzlich beispielsweise in einer Größenordnung erfolgen, in der in der Vorreinigung dem System Wasser zugeführt wird. Die im Phasentrenner abgeschiedene wässrige Phase weist üblicherweise einen Gehalt an organischen Inhaltsstoffen auf. Es kann daher vorteilhaft sein, dieses Wasser einer Form der Entsorgung zuzuführen, die diesen Gehalt an organischen Stoffen ausnutzt.

So kann es beispielsweise vorteilhaft sein, wenn ein derart mit organischen Stoffen belastetes Wasser im Rahmen eines Schwefelsäurespaltverfahrens dem Brennraum zugemischt wird. Aufgrund der oxidierbaren Inhaltsstoffe kann so deren Brennwert, zumindest teilweise, noch genutzt werden. Darüber hinaus wird so eine möglicherweise kostspielige Entsorgung des mit organischen Stoffen belasteten Wassers oft vermieden.

### Hauptreinigung des Methacrylsäureesters

Zur Hauptreinigung des Methacrylsäureesters wird der rohe, vorgereinigte Methacrylsäureester einer erneuten Destillation unterzogen. Dabei wird der rohe Methacrylsäureester mit Hilfe einer Destillationskolonne von seinen hochsiedenden Bestandteilen befreit und so ein Reinmethacrylsäureester erhalten. Hierzu wird der rohe Methacrylsäureester in einer dem Fachmann bekannten Weise in die untere Hälfte einer Destillationskolonne eingebracht.

Die Destillationskolonne kann grundsätzlich einer beliebigen, dem Fachmann geeignet erscheinenden Ausführung entsprechen. Es hat sich jedoch für die Reinheit des erhaltenen Produkts in vielen Fällen als vorteilhaft herausgestellt, wenn die Destillationskolonne mit einer oder mehreren Packungen betrieben wird, die den folgenden Vorgaben entspricht:
Zum einen sollen sich in den Kolonnen genauso wie in den anderen mit Methacrylsäureester durchströmten Leitungen möglichst wenig so genannte "Toträume" bilden. Die Toträume führen zu einer vergleichsweise langen Verweilzeit der Methacrylsäureester, die deren Polymerisation begünstigen. Dieses führt wiederum zu kostspieligen Produktionsunterbrechungen und Reinigungen der entsprechenden mit Polymer zugesetzten Teile. Der Bildung von Toträumen kann unter anderem dadurch begegnet werden, dass sowohl durch Auslegung als auch durch eine geeignete Betriebsweise der Kolonnen, diese stets mit einer ausreichenden Menge von Flüssigkeit beladen werden, so dass eine ständige Umspülung der Kolonnen und insbesondere der Kolonneneinbauten wie Packungen erreicht wird.

Im Rahmen der Reinigung des Methacrylsäureesters werden dessen hochsiedende Bestandteile durch Destillation vom Produkt getrennt. Hierzu wird der Kolonnensumpf mit Dampf beheizt. Die Sumpftemperatur beträgt dabei vorzugsweise 50 bis 80 °C, insbesondere 60 bis 75 °C bei Wandtemperatur von weniger als 120 °C.

Das im Kolonnensumpf anfallende Material wird vorzugsweise kontinuierlich abgeführt und über einen Wärmetauscher oder eine Kaskade von mehreren Wärmetauschern auf eine Temperatur in einem Bereich von 40 bis 80 °C, vorzugsweise 40 bis 60 °C und besonders bevorzugt in einem Bereich von 50 bis 60 °C abgekühlt.

Dieses Material, das überwiegend Methacrylsäureester, Hydroxyisobuttersäureester, Methacrylsäure und Stabilisatorkomponenten enthält, wird anschließend über einen Lagerbehälter, beispielsweise entsorgt oder einer anderweitigen Verwendung zugeführt. Es hat sich in vielen Fällen als vorteilhaft erwiesen, wenn das im Kolonnensumpf gewonnene Material in die Veresterungsreaktion zurückgeführt wird. Beispielsweise wird dabei das Material aus dem Kolonnensumpf in den ersten Veresterungskessel rückgeführt. Daraus ergibt sich der Vorteil, dass im Hinblick auf eine möglichst wirtschaftliche Fahrweise und eine möglichst hohe Ausbeute im Kolonnensumpf enthaltene, höhersiedende Verbindungen in die Veresterungsreaktion zurückgeführt werden.

Am Kolonnenkopf wird der destillativ aufgereinigte Methacrylsäureester entnommen und über einen Wärmetauscher oder eine Kaskade von zwei oder mehr Wärmetauschern gekühlt. Dabei kann die Wärme der Brüden durch wassergekühlte Wärmetauscher oder durch solegekühlte Wärmetauscher oder durch eine Kombination aus beidem abgeführt werden. Es hat sich in einigen Fällen bewährt, wenn die Brüden aus der Destillationskolonne in zwei oder mehr parallel geschaltete Wärmetauscher überführt werden, die mittels Wasserkühlung betrieben werden. Die nicht kondensierten Anteile aus den wassergekühlten Wärmetauschern können beispielsweise in einen solegekühlten Wärmetauscher oder eine Kaskade von zwei oder mehr solegekühlten Wärmetauschern eingeleitet werden, die seriell oder parallel angeordnet sein können. Die aus den Wärmetauschern erhältlichen Kondensate werden in einen Sammelbehälter eingeleitet und mittels einer Pumpe über einen weiteren Wärmetauscher oder eine Kaskade von zwei oder mehr weiteren Wärmetauschern einem Pufferbehälter zugeführt. Der Kondensatstrom wird dabei beispielsweise über eine Kaskade von einem oder zwei wassergekühlten Wärmetauschern und einem oder zwei solegekühlten Wärmetauschern auf eine Temperatur in einem Bereich von 0 bis 20 °C, vorzugsweise 0 bis 15 °C und besonders bevorzugt in einem Bereich von 2 bis 10 °C heruntergekühlt.

Dem Kondensatstrom wird ein Teilstrom entnommen, der über den Kolonnenkopf in die Destillationskolonne zurückgeführt wird. Die Einspeisung des Kondensatstromes in den Kolonnenkopf kann dabei grundsätzlich auf beliebige Weise z.B. über Verteiler erfolgen. Es kann jedoch vorteilhaft sein, wenn ein Teil des Kondensatstromes oberhalb des Kolonnenkopfes in die Brüdenleitung eingespeist, beispielsweise eingesprüht, wird. Weiterhin ist es bevorzugt, dass mit dieser Einspeisung Stabilisator in den Kolonnenkopf eingetragen wird.

Ein weiterer Teilstrom des zur Rückführung in die Kolonne vorgesehenen Kondensates kann beispielsweise vor Einbringung in die Brüdenleitung abgezweigt und direkt in den Kolonnenkopf eingebracht werden. Auch hier ist es bevorzugt, dass mit dieser Einspeisung Stabilisator in den Kolonnenkopf eingetragen wird. Die Einbringung in den Kolonnenkopf kann dabei beispielsweise dergestalt geschehen, dass das Innere des Kolonnenkopfes mit dem Kondensat so besprüht wird, dass sich keine Ruhezonen im Kolonnenkopf ausbilden können, an denen eine Polymerisation des Methacrylsäureesters erfolgen kann. Es kann darüber hinaus vorteilhaft sein, wenn einem Kondensatteilstrom, der in die Kolonne zurückgeführt wird, ein Stabilisator zur Verhinderung von Polymerisation zugefügt wird. Dies kann beispielsweise dadurch geschehen, dass dem zum Besprühen des Kolonnenkopfes vorgesehenen Kondensatteilstrom eine entsprechende Menge an Polymerisationsinhibitor als Stabilisator zugefügt wird. Dabei hat es sich in einigen Fällen als vorteilhaft erwiesen, wenn der Kondensatteilstrom nach der Zugabe des Stabilisators, aber vor dem Eintritt in den Kolonnenkopf eine geeignete Mischvorrichtung, vorzugsweise einen Statikmischer durchläuft, um eine möglichst uniforme Verteilung des Stabilisators im Kondensatteilstrom zu erzielen.

Die im Rahmen des Reinigungsverfahrens anfallenden, nicht kondensierbaren gasförmigen Stoffe werden beispielsweise der Entsorgung zugeführt.

Das im Pufferbehälter befindliche Rohprodukt wird mit Hilfe eines Solekühlers auf einer Temperatur von 0 bis 20 °C, vorzugsweise 0 bis 15 °C und besonders bevorzugt in einem Bereich von 2 bis 10 °C gehalten.

Um ggf. weitere Verunreinigungen aus dem Produkt zu entfernen und zur Reinstmethacrylsäureestern zu gelangen, kann das Produkt noch einer adsorptiven Reinigungsstufe unterzogen werden. Es hat sich dabei beispielsweise bewährt, wenn das Reinprodukt ganz oder zumindest ein Teil des Reinproduktes mit Hilfe eines Molekularsiebes weiter gereinigt wird. Besonders saure Verunreinigungen, insbesondere im Rahmen des Herstellungsverfahrens gebildete Ameisensäure, kann so auf einfache Weise aus dem Produktstrom entfernt werden. Dabei hat es sich darüber hinaus in einigen Fällen bewährt, wenn der Produktstrom nach dem Durchlaufen der adsorptiven Reinigungsstufe noch einen oder mehrere Filter durchläuft, um ggf. im Produkt enthaltene Feststoffe zu entfernen.

Die im Rahmen der Aufarbeitung anfallenden Stoffströme umfassen überwiegend polymerisierbare Verbindungen. Um die Ausbildung von Ruhezonen zu unterbinden, hat es sich auch im Fall des hier beschriebenen Verfahrens als vorteilhaft herausgestellt, wenn die Teile der Anlage, die mit Methacrylsäureester in Berührung kommen, ständig mit Methacrylsäureester überströmt sind. Im Rahmen einer weiteren Ausführungsform des hier vorgestellten Verfahrens wird daher ein Teilstrom an Methacrylsäureester nach dem Pufferbehälter, jedoch vor der adsorptiven Reinigungsstufe entnommen, um die Kopfbereiche derjenigen Wärmetauscher zu überspülen, welche die aus der Destillationskolonne stammenden Brüden aufnehmen.

Insgesamt hat es sich im Rahmen der vorliegenden Erfindung als vorteilhaft erwiesen, wenn der Verbund aus Vorreinigung und Hauptreinigung derart gestaltet wird, dass
- bei der Vorreinigung Stoffe abgetrennt werden, die einen niedrigeren Siedepunkt aufweisen als der Methacrylsäureester und diese Stoffe anschließend durch Kühlung kondensiert werden, wobei nicht kondensierte Reststoffe in der Gasphase verbleiben,
- bei der Hauptreinigung Stoffe abgetrennt werden, die einen höheren Siedepunkt aufweisen als der Methacrylsäureester und dieser durch Kühlung kondensiert wird, wobei nicht kondensierte Reststoffe in der Gasphase verbleiben und
- nicht kondensierte gasförmige Reststoffe aus der Vorreinigung und nicht kondensierte gasförmige Reststoffe aus der Hauptreinigung einer gemeinsamen Nachkondensation unterzogen werden.

Ein bei einer solchen gemeinsamen Nachkondensation anfallendes Kondensat kann vorteilhafterweise einer Phasentrennung unterzogen werden, wobei sich eine wässrige und eine organische Phase bilden können. In diesem Fall kann beispielsweise die wässrige Phase ganz oder teilweise in die Veresterung zurückgeführt werden oder die organische Phase ganz oder teilweise in die Vorreinigung zurückgeführt werden oder beides.

Das im Rahmen der Reinigungsstufe insgesamt gewonnene Produkt wird anschließend mit einer Temperatur in einem Bereich von -5 bis 20 °C, vorzugsweise 0 bis 15 °C und besonders bevorzugt in einem Bereich von 2 bis 10 °C der Reinigungsstufe entnommen.

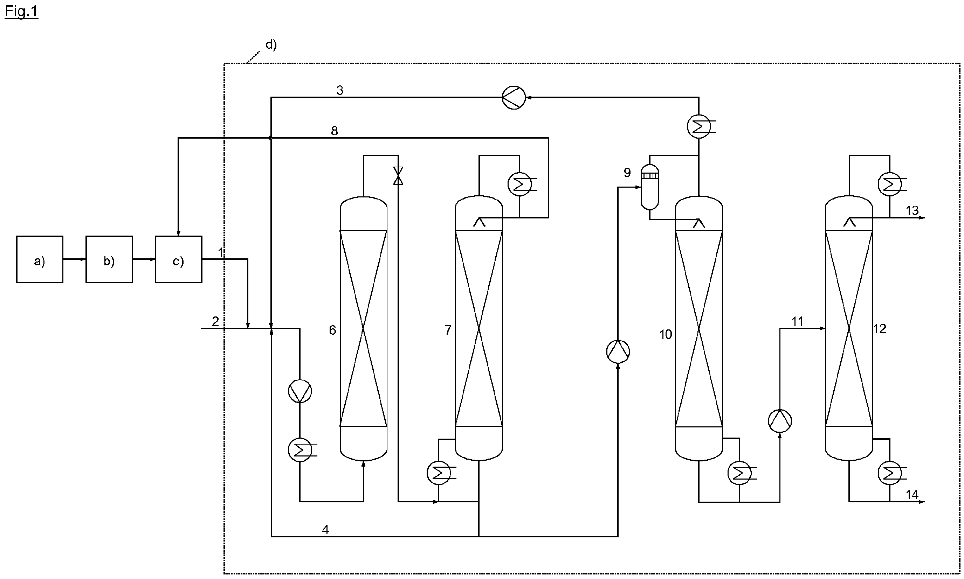

### Beschreibung der Zeichnung (Fig. 1)

### Schritt d) Hydrolyse der Methacrylsäureester zu Methacrylsäure

Das über die Vorstufen synthetisierte Methylmethacrylat (1) bzw. der jeweilige Methacrylsäureester und das zur Hydrolyse zu Methacrylsäure erforderliche Wasser (2) werden in die Anlage zugeführt. Nach der Eindosierung werden die Edukte mit den Kreislaufströmen (3) und (4) vereinigt. Das Gemisch wird einem Wärmetauscher (5) zugeführt, durch den die Reaktionsmischung auf die gewünschte Reaktionstemperatur gebracht wird. Anschließend wird das Reaktionsgemisch einem Festbettrohrreaktor (6) zugeführt. Der Festbettrohrreaktor enthält heterogene Katalysatoren. Die Katalysatoren werden ausgewählt aus der Gruppe der Zeolithe, lonenaustauscherharze oder amorphen Säurekatalysatoren. Besonders bevorzugt sind kationische lonenaustauscherharze, insbesondere der Ionenaustauscher der Fa. Lanxess AG, Typ: Lewatit K2431.

In Abhängigkeit von der Polymerisationsneigung des eingesetzten Monomergemisches, der Art des Katalysators und/oder der Größe des Katalysatorbettes, erfolgt die Durchströmung des Katalysatorbettes von oben oder unten. Bevorzugt wird von unten angeströmt und das Reaktionsgemisch über den Katalysatorbettboden zugeführt.

An diesem Katalysator findet die Hydrolyse von Methylmethacrylat bzw. des jeweiligen Methacrylats zu Methacrylsäure statt. Im Folgenden wird beispielhaft die Hydrolyse von Methylmethacrylat zu Methacrylsäure beschrieben. Entsprechende Adaptionen für andere Methacrylsäureester können in der dem Fachmann bekannten Weise durchgeführt werden. Es wurde gefunden, dass eine geringe H₂O-Konzentration im Reaktionsstrom, <10 Gew.-%, vorzugsweise < 5 Gew.-%, insbesondere < 1 Gew.-% zu einer deutlichen Verringerung des Dampfbedarfes führt, der zur Abtrennung des Methanol/Methylmethacrylat-Azeotrops erforderlich ist.

Da jedoch der Umsatz und die Raum-Zeit-Ausbeute bei einer Verringerung der H₂O-Konzentration sinken, muss das Optimum der H₂O-Konzentration eingestellt werden.

Um zu gewährleisten, dass auch bei Reaktionstemperaturen oberhalb des Siedepunktes der Mischung eine homogene flüssige Reaktionsphase vorliegt, kann der Reaktor unter einem geringen Überdruck von ca. 2 bis 4 bar betrieben werden.

Das bei der Reaktion entstehende Methanol wird als Azeotrop mit Methylmethacrylat in einer nachgeschalteten Rektifizierkolonne (7) als Kopfstrom (8) abgetrennt. Der Großteil des Sumpfstromes kann als Kreislaufstrom (4) zurückgeführt werden. Ein Teil wird zur Leichtsieder-Abtrennung über eine Flash-Kammer (9) in eine Vakuum-Rektifizierkolonne (10) geführt werden. Hierin werden Methylmethacrylat und H₂O sowie Restmethanol über den Kolonnenkopf abgetrennt (3) und können wieder zurückgeführt werden.

Die im Kolonnensumpf angereicherte Methacrylsäure kann als Rohmethacrylsäure abgetrennt werden(11). Vorzugsweise wird die Methacrylsäure in einer weiteren nachgeschalteten Vakuum-Rektifizierkolonne (12) von den enthaltenen Hochsiedern (14) (Stabilisatoren, Nebenprodukte) abgetrennt und über den Kolonnenkopf als reine Methacrylsäure (13) gewonnen. Die so erhaltene Methacrylsäure weist eine Reinheit von ≥ 99,5 % auf.

Zur weiteren Optimierung des Gesamtprozesses kann das Methanol/Methylmethacrylat-Gemisch (8) in den Verfahrensschritt c) zurückgeführt werden. Üblicherweise hat das Gemisch von Methanol zu Methylmethacrylat eine gemäß Literatur beschriebene Zusammensetzung in der Nähe des Azeotrops, in der betrieblichen Praxis eine Zusammensetzung von mindestens 60 % Methanol, vorzugsweise mindestens 75 % Methanol. Das im Gemisch befindliche Methanol kann wieder zur Veresterung von Methacrylamid eingesetzt werden.

### Bezugszeichenliste

- 1: Methylmethacrylat (MMA)-Feed
- 2: Wasserfeed
- 3: Kreislaufstrom von Rektifizierkolonne zur Methanolabtrennung
- 4: Kreislaufstrom von Vakuum-Rektifizierkolonne zur Leichtsiederabtrennung
- 5: Wärmeaustauscher
- 6: Reaktor
- 7: Rektifizierkolonne zur Methanolabtrennung
- 8: Kopfstrom der Rektifizierkolonne zur Methanolabtrennung
- 9: Flashkammer
- 10: Vakuum-Rektifizierkolonne zur Leichtsiederabtrennung
- 11: Sumpfstrom der Vakuum-Rektifizierkolonne zur Leichtsiederabtrennung
- 12: Vakuum-Rektifizierkolonne zur Methacrylsäureabtrennung
- 13: Methacrylsäurestrom
- 14: Hochsiederstrom

Die im Folgenden gegebenen Beispiele werden zur besseren Veranschaulichung der vorliegenden Erfindung gegeben, sind jedoch nicht dazu gedacht, die Erfindung auf die hierin offenbarten Merkmale zu beschränken.

### Beispiele

### Beispiel 1

Acetoncyanhydrin wird aus der basisch katalysierten Umsetzung von Cyanwasserstoff mit Aceton gewonnen (a). Nach der Hydrolyse zu Methacrylamid (b) wird in Gegenwart von Methanol zu Methylmethacrylat umgeestert (c).

12,7 kg/h Methylmethacrylat (1) werden mit 2,1 kg/h Wasser (2) zusammengeführt.. Das Reaktionsgemisch wird zusammen mit 15,4 kg/h Kreislaufstrom (3) mit 500 kg/h Kreislaufstrom (4) durch einen Wärmetauscher (5) auf eine Temperatur von 110 °C gebracht und von unten durch den mit dem kationischen lonenaustauscherharz Lewatit K2431 der Fa. Lanxess AG gefüllten Reaktor (6) geführt. An diesem kationischen lonenaustauscherharz findet die Hydrolyse von Methylmethacrylat zu Methacrylsäure statt. Die Umsetzung erfolgt bei einem Druck von 3 bar Überdruck am Reaktorausgang gemessen.

Das bei der Reaktion entstehende Methanol wird als Azeotrop mit Methylmethacrylat in einer nachgeschalteten Rektifizierkolonne (7) als Kopfstrom mit 4,8 kg/h abgetrennt (8) und gelangt zur Methylmethacrylatherstellung (c) zurück. Aus dem Kolonnensumpf der Rektifizierkolonne (7) werden 500 kg/h Kreislaufstrom (3) zurückgeführt, die verbleibenden 25,4 kg/h werden aus dem Kolonnensumpf zur Leichtsieder-Abtrennung über eine Flash-Kammer (9) in eine weitere Vakuum-Rektifizierkolonne (10) geführt. Hierin gehen die enthaltenen Leichtsieder Methylmethacrylat, Wasser sowie Restmethanol über den Kolonnenkopf wieder als Kreislaufstrom mit 15,4 kg/h (3) zurück. Der im Kolonnensumpf angereicherte Methacrylsäurestrom von 10,0 kg/h (11) wird in einer weiteren nachgeschalteten Vakuum-Rektifizierkolonne (12) aufgereinigt und über Kopf abgetrennt (13). Hochsieder werden über den Sumpf ausgeschleust (14).

### Beispiel 2

Es wurde der Schwefelsäurebedarf für die konventionelle Methacrylsäureproduktion in einem Methylmethacrylat/Methacrylsäure-Verbund ermittelt. Dazu wurde der Schwefelsäurebedarf für das erfindungsgemäße Verfahren zur Herstellung von Methacrylsäure ermittelt.

Die konventionelle Methacrylsäure wurde über die Verseifung des Amids dargestellt.

| | konventinelle Methacrylsäure Herstellung | erfindungsgemäße Methacrylsäure Herstellung | Differenz |
|---|---|---|---|
| Verbrauch H₂SO₄ | 178 kt | 174 kt | 4 kt |
| Anfall Abfallsäure | 255 kt | 247 kt | 8 kt |
| Wassergehalt Abfallsäure | 27 % | 17,5 % | 9,5 % |
| Wassergehalt MAS | 1500-2000 ppm | <1000 ppm | 500-1000 ppm |

Mit dem erfindungsgemäßen Verfahren werden große Mengen Schwefelsäure eingespart. Zusätzlich kann die Menge an Abfallsäure stark reduziert werden.

Die Abfallsäure besteht größtenteils aus Schwefelsäure und Ammoniumsulfat. Dieses Gemisch wird in eine Schwefelsäurespaltanlage eingespeist.

## Patentansprüche

1. Verfahren zur Herstellung von Methacrylsäure aufweisend folgende Schritte:
a) Bereitstellung von Acetoncyanhydrin
b) Umsetzung von Acetoncyanhydrin zu Methacrylamid
c) Veresterung von Methacrylamid in Gegenwart von Alkoholen zu Methacrylsäureestern
d) Hydrolyse der Methacrylsäureester zu Methacrylsäure mittels heterogener Katalysatoren
und einem Methacrylsäureester/H₂O Verhältnis des Eduktstromes zwischen 0,5 und 5.

2. Verfahren zur Herstellung von Methacrylsäure gemäß Anspruch 1, **dadurch gekennzeichnet, dass** Katalysatoren ausgewählt aus der Gruppe der Zeolite, lonenaustauscherharze und amorphen Säurekatalysatoren verwendet werden, besonders bevorzugt kationische lonenaustauscherharze.

3. Verfahren zur Herstellung von Methacrylsäure gemäß Anspruch 1, **dadurch gekennzeichnet, dass** das Katalysatorbett von unten oder oben durchströmt wird, bevorzugt von unten vom Katalysatorbettboden aus.

4. Verfahren zur Herstellung von Methacrylsäure gemäß Anspruch 1, **dadurch gekennzeichnet, dass** die Hydrolyse bei 50-200 °C durchgeführt wird, bevorzugt bei 70-150 °C, besonders bevorzugt bei 90-120 °C, insbesondere 100-110°C.

5. Verfahren zur Herstellung von Methacrylsäure gemäß Anspruch 1, **dadurch gekennzeichnet, dass** die Hydrolyse bei Überdruck durchgeführt wird, bevorzugt bei 0,1-9 bar Überdruck, besonders bevorzugt bei 2-4 bar Überdruck.

6. Verfahren zur Herstellung von Methacrylsäure gemäß Anspruch 1, **dadurch gekennzeichnet, dass** das Methacrylsäureester/H₂O Verhältnis des Eduktstromes zwischen 1 und 4, bevorzugt zwischen 1,5 und 3 liegt.

7. Verfahren zur Herstellung von Methacrylsäure gemäß Anspruch 1, **dadurch gekennzeichnet, dass** in Schritt d) die Verweilzeit 10-120 min beträgt, bevorzugt 30-90 min, besonders bevorzugt 45-75 min.

8. Verfahren zur Herstellung von Methacrylsäure gemäß Anspruch 1, **dadurch gekennzeichnet, dass** als Methacrylsäureester Methylmethacrylat verwendet wird, dass das in Schritt d) bei der Reaktion entstehende Methanol als Azeotrop mit Methylmethacrylat in einer nachgeschalteten Rektifizierkolonne als Kopfstrom abgetrennt wird, und das Methanol/Methylmethacrylat Gemisch dem Schritt c) zugeführt wird.

## Claims

1. Process for preparing methacrylic acid, comprising the following steps:
a) provision of acetone cyanohydrin
b) conversion of acetone cyanohydrin to methacrylamide
c) esterification of methacrylamide in the presence of alcohols to give methacrylic esters
d) hydrolysis of the methacrylic esters to give methacrylic acid,
by means of heterogeneous catalysts
and a methacrylic esters/H₂O ratio of the reactant stream between 0.5 and 5.

2. Process for preparing methacrylic acid according to Claim 1, **characterized in that** catalysts selected from the group of the zeolites, ion exchange resins and amorphous acid catalysts are used, more preferably cationic ion exchange resins.

3. Process for preparing methacrylic acid according to Claim 1, **characterized in that** the flow through the catalyst bed is from the bottom or from the top, preferably from the bottom proceeding from the catalyst bed base.

4. Process for preparing methacrylic acid according to Claim 1, **characterized in that** the hydrolysis is performed at 50-200°C, preferably at 70-150°C, more preferably at 90-120°C, especially 100-110°C.

5. Process for preparing methacrylic acid according to Claim 1, **characterized in that** the hydrolysis is performed at elevated pressure, preferably at 0.1-9 bar gauge, more preferably at 2-4 bar gauge.

6. Process for preparing methacrylic acid according to Claim 1, **characterized in that** the methacrylic esters/H₂O ratio of the reactant stream is between 1 and 4, preferably between 1.5 and 3.

7. Process for preparing methacrylic acid according to Claim 1, **characterized in that** the residence time in step d) is 10-120 min, preferably 30-90 min, more preferably 45-75 min.

8. Process for preparing methacrylic acid according to Claim 1, **characterized in that** as methacrylic ester methyl methacrylate is used, **in that** the methanol formed in the reaction in step d) is removed as a top stream in a downstream rectifying column as an azeotrope with methyl methacrylate, and the methanol/methyl methacrylate mixture is supplied to step c).

## Revendications

1. Procédé de fabrication d'acide méthacrylique comprenant les étapes suivantes :
a) la préparation d'acétone-cyanhydrine,
b) la transformation de l'acétone-cyanhydrine en méthacrylamide,
c) l'estérification du méthacrylamide en présence d'alcools en esters de l'acide méthacrylique,
d) l'hydrolyse des esters de l'acide méthacrylique en acide méthacrylique au moyen de catalyseurs hétérogènes, et à un rapport esters de l'acide méthacrylique/H₂O du courant de réactifs compris entre 0,5 et 5.

2. Procédé de fabrication d'acide méthacrylique selon la revendication 1, **caractérisé en ce que** des catalyseurs choisis dans le groupe constitué par les zéolithes, les résines échangeuses d'ions et les catalyseurs acides amorphes sont utilisés, de manière particulièrement préférée des résines échangeuses d'ions cationiques.

3. Procédé de fabrication d'acide méthacrylique selon la revendication 1, **caractérisé en ce que** le lit catalytique est traversé depuis le bas ou depuis le haut, de préférence depuis le bas depuis le fond du lit catalytique.

4. Procédé de fabrication d'acide méthacrylique selon la revendication 1, **caractérisé en ce que** l'hydrolyse est réalisée à une température de 50 à 200 °C, de préférence à une température de 70 à 150 °C, de manière particulièrement préférée à une température de 90 à 120 °C, notamment à une température de 100 à 110 °C.

5. Procédé de fabrication d'acide méthacrylique selon la revendication 1, **caractérisé en ce que** l'hydrolyse est réalisée à une surpression, de préférence à une surpression de 0,1 à 9 bar, de manière particulièrement préférée à une surpression de 2 à 4 bar.

6. Procédé de fabrication d'acide méthacrylique selon la revendication 1, **caractérisé en ce que** le rapport esters de l'acide méthacrylique/H₂O du courant de réactifs est compris entre 1 et 4, de préférence entre 1,5 et 3.

7. Procédé de fabrication d'acide méthacrylique selon la revendication 1, **caractérisé en ce qu'**à l'étape d), le temps de séjour est de 10 à 120 minutes, de préférence de 30 à 90 minutes, de manière particulièrement préférée de 45 à 75 minutes.

8. Procédé de fabrication d'acide méthacrylique selon la revendication 1, **caractérisé en ce que** du méthacrylate de méthyle est utilisé en tant qu'ester de l'acide méthacrylique, **en ce que** le méthanol formé lors de la réaction à l'étape d) est séparé en tant qu'azéotrope avec du méthacrylate de méthyle dans une colonne de rectification raccordée en aval en tant que courant de tête, et le mélange méthanol/méthacrylate de méthyle est introduit dans l'étape c).
